# EUROPEAN PATENT SPECIFICATION

(11) **EP 3 130 291 B1**
(45) Date of publication and mention of the grant of the patent: **27.05.2020**
(21) Application number: 16169842.8
(22) Date of filing: 10.01.2008
(51) Int. Cl.: A61B 17/072

(54) **IMPROVED BUTTRESS MATERIAL FOR USE WITH A SURGICAL STAPLER**
VERBESSERTES VERSTEIFUNGSMATERIAL ZUR VERWENDUNG MIT EINEM CHIRURGISCHEN KLAMMERGERÄT
MATÉRIAU DE RENFORT AMÉLIORÉ POUR UTILISATION AVEC UNE AGRAFEUSE CHIRURGICALE

(30) Priority: 11.01.2007 US 652423
(43) Date of publication of application: 15.02.2017
(62) Divisional of application: 08250112.3
(73) Proprietor: Ethicon LLC, 00969 Guaynabo (PR)
(72) Inventor: SHELTON IV, Frederick E., New Vienna, OH Ohio 45159 (US)
(74) Representative: Carpmaels & Ransford LLP

(56) References cited:
- EP-A2- 0 511 470
- EP-A2- 1 256 317
- EP-A2- 1 621 141
- WO-A1-96/22055
- WO-A1-98/17180
- WO-A1-02/060328
- WO-A2-2006/044490
- US-A- 5 397 324
- US-B1- 6 273 897

## Description

### BACKGROUND

### 1. Field of the Invention

The present invention generally relates to surgical staplers, and, more particularly, to surgical staplers having a curved end-effector.

### 2. Description of the Related Art

As known in the art, surgical staplers are often used to deploy staples into soft tissue to reduce or eliminate bleeding from the soft tissue, especially as the tissue is being transected, for example. Surgical staplers, such as an endocutter, for example, often comprise an end-effector which is configured to secure the soft tissue between first and second jaw members. The first jaw member often includes a staple cartridge which is configured to removably store staples therein and the second jaw member often includes an anvil. In use, the staples are typically deployed from the staple cartridge by a driver which traverses a channel in the staple cartridge. The driver causes the staples to be deformed against the anvil and secure layers of the soft tissue together. Often, as known in the art, the staples are deployed in several staple lines, or rows, in order to more reliably secure the layers of tissue together. The end-effector may also include a cutting member, such as a knife, for example, which is advanced between two rows of the staples to resect the soft tissue after the layers of the soft tissue have been stapled together.

The end-effectors of previous endocutters are often configured to deploy staples in straight lines. During many surgical techniques, such as the resection of stomach tissue, for example, such a linear deployment is often preferred. During these techniques, the end-effector is typically inserted through a cannula to access the surgical site and, as a result, it is often desirable for the end-effector to have a linear configuration that can be aligned with an axis of the cannula before the end-effector is inserted therethrough. However, in some circumstances, end-effectors having such a linear configuration are somewhat difficult to use. More particularly, for example, when the end-effector must be placed adjacent to or against a cavity wall, such as the thoracic cavity wall, for example, it is often difficult for the surgeon to position a jaw of the end effector behind delicate or fragile tissue which is proximal to and/or attached to the cavity wall. Furthermore, even if the surgeon is successful in positioning a jaw behind the tissue, owing to the linear configuration of the end-effector, the surgeon may not be able to see the distal end of the end-effector.

In some circumstances, endocutters having a curved end-effector have been used for accessing, stapling and transecting tissue. These end-effectors typically include curved anvils and staple cartridges which co-operate to deploy the staples in curved rows. To deploy the staples in this manner, the staple driver and the cutting member can be moved through a curved path by a flexible drive member. However, owing to the amount of force that is typically transmitted through the flexible drive member, the drive member may buckle or otherwise deform in an unsuitable manner. Furthermore, previous curved end-effectors are configured such that the distal ends of the jaw members are the last portions of the jaw members to contact the soft tissue. As a result, tissue may escape from between the jaw members before the jaw members are completely closed. What is needed is an improvement over the foregoing.

US 5,397,324 A discloses a surgical stapler instrument and method for enhancing blood vessel hemostasis. Lips are provided to hold pads on the stapler cartridge. EP 1 621 141 A2 discloses a surgical instrument for being endoscopically or laparoscopically inserted to a surgical site.

### SUMMARY

The invention is defined by appended claim 1. Preferred embodiments are defined in the dependent claims.

The surgical stapler of the present invention can further include the following features:
a second lip which at least partially extends along said first end.
a second lip which at least partially extends along said second end.
that said surface further includes a second edge extending between said first end and said second end, wherein said second edge is at least partially defined by a second radius of curvature, and wherein said surgical stapler further includes a second lip which at least partially extends along said second edge.
adhesive for securing said piece of buttress material to said surface.
a second surface configured to receive a second piece of buttress material thereon and a second lip extending along the periphery of said second surface, and wherein said second lip is configured to reduce relative movement between said second piece of buttress material and said second surface.
that said anvil includes said second surface and said second lip, and wherein said anvil further comprises a third surface configured to receive a third piece of buttress material thereon and a third lip extending along the periphery of said third surface.

### BRIEF DESCRIPTION OF THE FIGURES

The above-mentioned and other features and advantages of this invention, and the manner of attaining them, will become more apparent and the invention itself will be better understood by reference to the following description of embodiments of the invention taken in conjunction with the accompanying drawings, wherein:
Fig. 1 is a schematic of an endocutter being used to transect and staple tissue;
Fig. 2 is a partial cut-away view of the endocutter of Fig. 1;
Fig. 3 is a partial cross-sectional view of the endocutter of Fig. 2 taken along line 3-3 in Fig. 2;
Fig. 4 is a perspective cut-away view of the endocutter of Fig. 2;
Fig. 5 is a bottom view of the anvil of the endocutter of Fig. 2;
Fig. 6 is a schematic view of staples being deployed from the staple cartridge of the endocutter of Fig. 2 by a staple driver;
Fig. 7 is a schematic view of staples being deployed from the staple cartridge of Fig. 2 where the staple driver has been advanced within the staple cartridge with respect to its position in Fig. 6;
Fig. 8 is a perspective view of the cutting member and drive bar of the endocutter of Fig. 2;
Fig. 9 is a schematic of an opened thoracic cavity;
Fig. 10 is a schematic of an endocutter having a curved end-effector being positioned against the side wall of a thoracic cavity;
Fig. 11 is a perspective view of the endocutter of Fig. 10 illustrated in a closed configuration and positioned about a pulmonary artery;
Fig. 12 is a perspective view of the end-effector of the endocutter of Fig. 11;
Fig. 13 is a top view of the staple cartridge of the end-effector of Fig. 12;
Fig. 14 is a bottom view of the jaw configured to support the staple cartridge of Fig. 13;
Fig. 15 is a perspective view of the cutting member and staple driver of the endocutter of Fig. 2;
Fig. 16 is a top view of the cutting member and staple driver of Fig. 15;
Fig. 17 is a top view of a cutting member and staple driver;
Fig. 18 is a perspective view of an endocutter having a curved end-effector;
Fig. 19 is a top view of the staple cartridge of the end-effector of Fig. 18;
Fig. 20 is a perspective view of an endocutter having a curved end-effector;
Fig. 21 is a top view of the staple cartridge of the end-effector of Fig. 20;
Fig. 22 is a perspective view of an endocutter having a curved end-effector;
Fig. 23 is a top view of the staple cartridge of the end-effector of Fig. 22;
Fig. 24 is a cross-sectional view of the end-effector of Fig. 12 taken along line 24-24 in Fig. 12;
Fig. 25 is a cross-sectional view of the end-effector of Fig. 12 after the drive bar has been advanced into the end-effector;
Fig. 26 is a schematic of the cutting member and drive bar of the endocutter of Figs. 24 and 25;
Fig. 27 is a perspective view of an endocutter having a curved end-effector configured to close in an asymmetric manner;
Fig. 28 is a cross-sectional view of the hinge connection between the jaws of the curved end-effector of Fig. 27 wherein the jaws are in an open configuration;
Fig. 29 is a cross-sectional view of the hinge connection of Fig. 28 wherein the jaws are in a partially closed configuration;
Fig. 30 is an end view of the curved end-effector of Fig. 27 illustrated in a partially closed configuration;
Fig. 31 is a cross-sectional view of the hinge connection of Fig. 28 wherein the end-effector is in a closed configuration;
Fig. 32 is an end view of the curved end-effector of Fig. 27 illustrated in a closed configuration;
Fig. 33 is a detail view of a first slot of the hinge connection of Fig. 28 that is configured to receive a first projection extending from the anvil and is also configured to define a first path for relative movement therebetween;
Fig. 34 is a detail view of a second slot of the hinge connection of Fig. 28 that is configured to receive a second projection extending from the anvil and is also configured to define a path for relative movement therebetween that is different than the first path;
Fig. 35 is a perspective view of an endocutter having a curved end-effector;
Fig. 36 is a side view of the endocutter of Fig. 35;
Fig. 37 is a schematic of the endocutter of Fig. 35 being used to transect a pulmonary artery;
Fig. 38 is a perspective view of an endocutter having a curved end-effector;
Fig. 39 is a perspective view of the staple cartridge of the end-effector of Fig. 38;
Fig. 40 is a side view of the end-effector of the endocutter of Fig. 39;
Fig. 41 is a partial cross-sectional view of the end-effector of the endocutter of Fig. 38;
Fig. 42 is a perspective view of the staple driver, cutting member and drive bar of Fig. 41;
Fig. 43 is a perspective view of the cutting member and drive bar of Fig. 41;
Fig. 44 is a perspective view of an endocutter having a curved staple cartridge and a curved anvil configured to retain buttress material thereon in accordance with an embodiment of the present invention;
Fig. 45 is a top view of the staple cartridge of Fig. 44 illustrating a piece of buttress material positioned thereon;
Fig. 46 is a bottom view of the anvil of Fig. 44 illustrating two pieces of buttress material positioned thereon;
Fig. 47 is a cross-sectional view of the end-effector of the endocutter of Fig. 44 taken along line 47-47 in Fig. 44;
Fig. 48 is a perspective view of an endocutter;
Fig. 49 is a cross-sectional view of the end effector of Fig. 48 taken along line 49-49 in Fig. 48; and
Fig. 50 is an enlarged cross-sectional view of the distal end of the end effector of Fig. 49.

Corresponding reference characters indicate corresponding parts throughout the several views. The exemplifications set out herein illustrate preferred embodiments of the invention, in various forms, and such exemplifications are not to be construed as limiting the scope of the invention in any manner.

### DETAILED DESCRIPTION

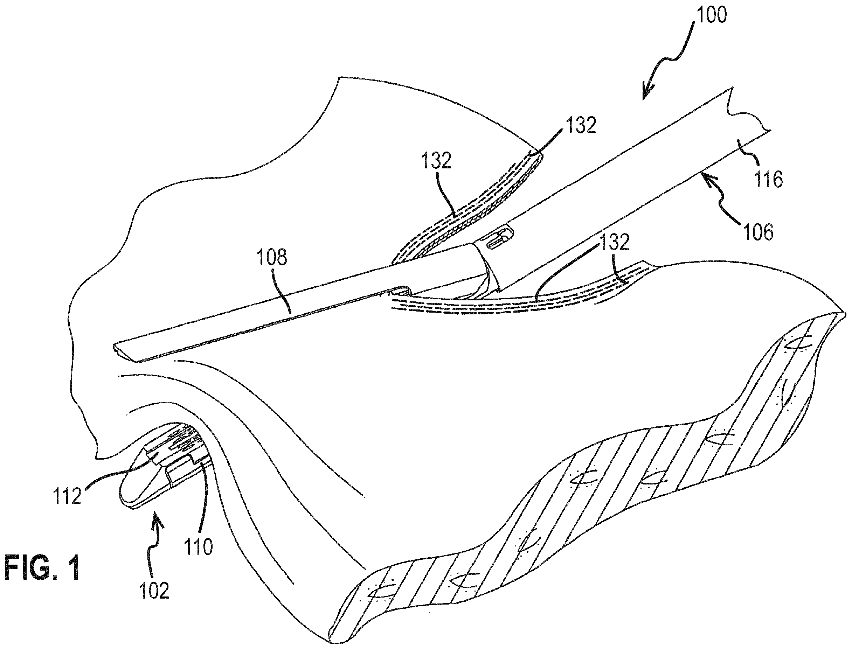

As known in the art, it is often necessary to resect tissue from a patient after the tissue has become necrotic or cancerous, for example. Frequently, blood vessels within the tissue are transected as the tissue is being cut. As a result, blood may flow from the blood vessels and complicate the surgery or endanger the patient. Often, a surgical stapler is used to secure and compress several layers of tissue together in order to substantially close the blood vessels. For example, referring to Fig. 1, a surgical stapler, such as an endocutter, can include devices which staple and then cut the tissue. As a result, the blood vessels can be substantially closed by the staples before the tissue is cut, thereby reducing bleeding therefrom.

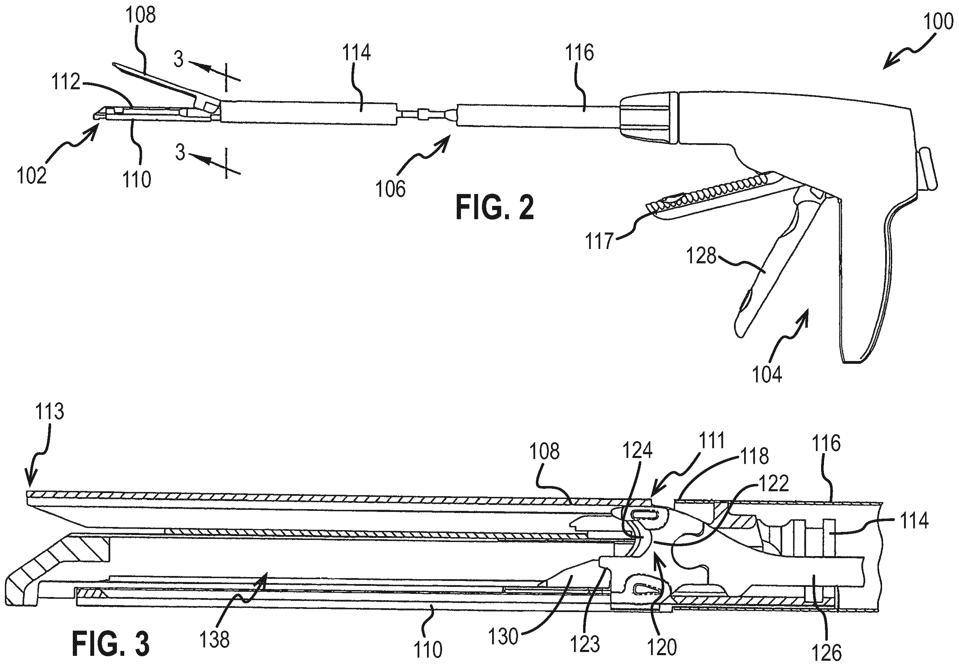

Referring to Figs. 1 and 2, endocutters, such as endocutter 100, for example, typically include an end-effector 102, a handle portion 104 (Fig. 2), and a shaft 106 extending therebetween. End-effector 102 includes first jaw 108 and second jaw 110 which can be configured in one of an open or a closed configuration. In their open configuration, jaws 108 and 110 can be configured to receive soft tissue therebetween, for example, allowing jaws 108 and 110 to be placed on opposite sides thereof. To close the jaws and secure the tissue therebetween, at least one of the jaws is moved against the tissue such that it holds the tissue against the opposing jaw. In the illustrated embodiment, jaw 108 is moved relative to jaw 110. Once closed, as known in the art, an anti-firing mechanism can be released allowing cutting member 120 to be advanced toward the tissue. Thereafter, as described in greater detail below, staples 132 can be deployed from staple cartridge 112 in jaw 110 to secure the layers of tissue together. Such mechanisms are described in greater detail in U.S. Patent No. 7,000,818.

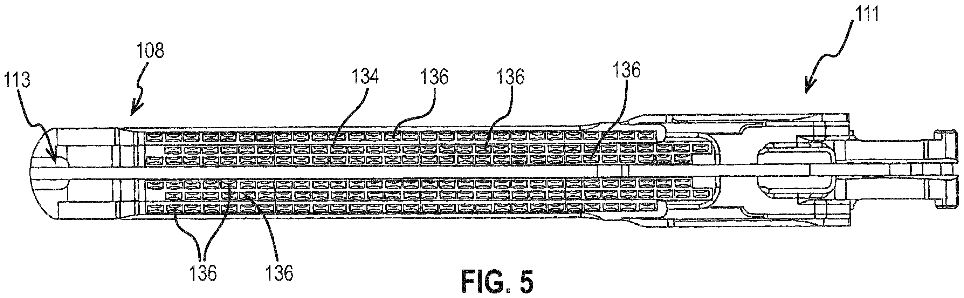

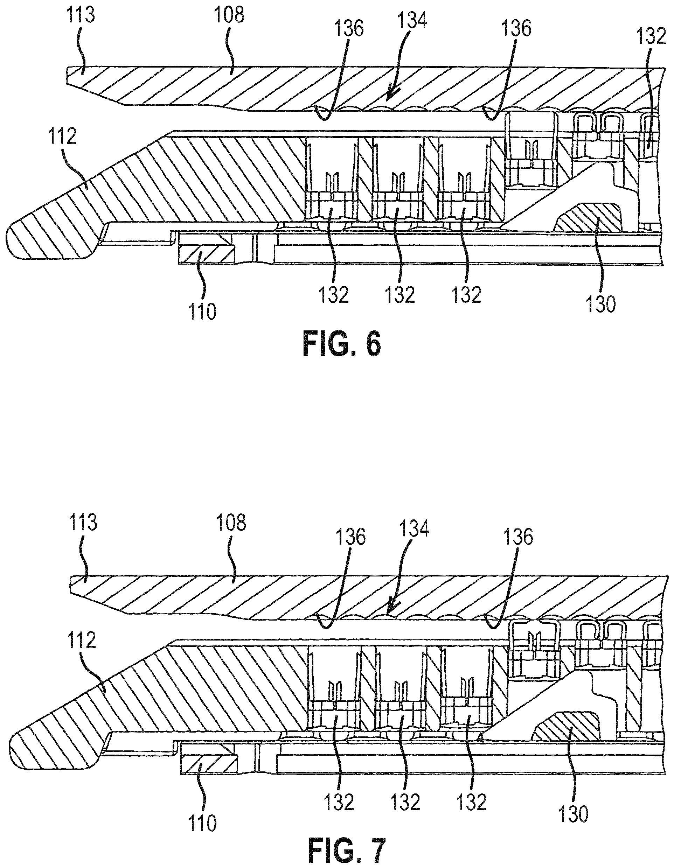

Referring to Figs. 3-4 and 6-8, cutting member 120 includes body 122 and cutting surface 124. Cutting member 120 is operably engaged with firing trigger 128 of handle portion 104 via drive bar 126 wherein the actuation of firing trigger 128 advances drive bar 126 and cutting member 120 toward the distal ends of jaws 108 and 110. In various embodiments, firing trigger 128 can activate a firing drive system which may be manually, electrically, or pneumatically driven. Cutting member body 122 further includes distal portion 123 which is configured to engage a staple driver 130 commonly supported within staple cartridge 112 and advance staple driver 130 therein. As staple driver 130 is advanced, staples 132 are lifted by driver 130 toward anvil 134. Referring to Fig. 5, anvil 134 includes pockets 136 which are configured to deform the legs of staples 132 and capture the layers of tissue therein in a known manner. In the present embodiment, as staple driver 130 is advanced, cutting member 120 is also advanced to resect the tissue after it has been stapled. In other embodiments, cutting member 120 can be configured to resect the tissue during or before the tissue has been stapled.

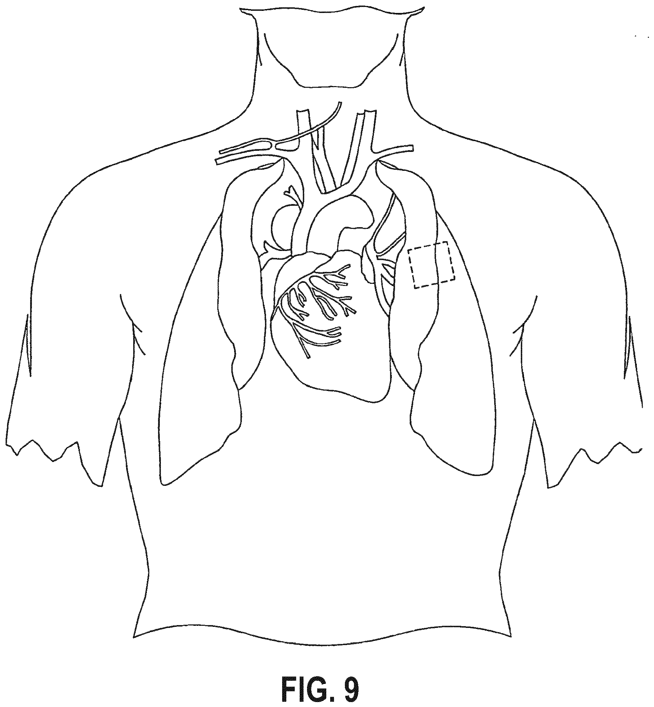

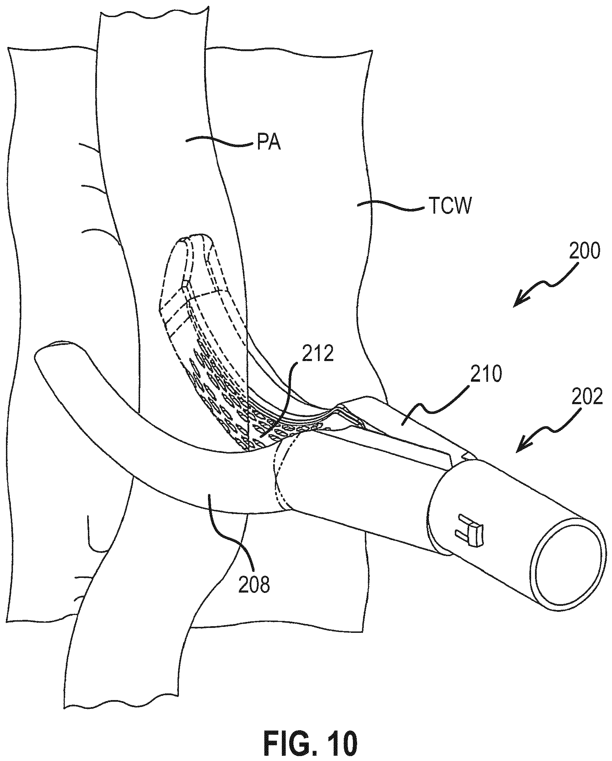

Referring to Figs. 1-7, the end-effector of many typical endocutters is linear, i.e., it is configured to deploy staples in straight lines. In these endocutters, drive bar 126 is configured to move cutting member 120 in a straight line and, accordingly, drive bar 126 is rigid such that it does not substantially deflect when the force to deploy the staples and transect the tissue is transmitted therethrough. In addition to the above, a variety of other drive arrangements are known for deploying staples in straight lines while resecting the tissue located between opposite lines of staples. However, it is often difficult to position such linear end-effectors in a surgical site. During at least one surgical technique, referring to Figs. 9 and 10, an endocutter is used to transect and staple a pulmonary artery (PA) during a partial or total pneumonectomy. During this technique, the end-effector is typically placed against the wall of the thoracic cavity (TCW) such that jaw 110, and staple cartridge 112, are positioned behind the pulmonary artery. However, as the wall of the thoracic cavity is typically curved, it is often difficult to position linear jaw 110 behind the pulmonary artery. Furthermore, even if the surgeon is successful in positioning a jaw behind the pulmonary artery, the surgeon, owing to the linear configuration of the end-effector, cannot readily see the end of the jaw as it is typically hidden behind the pulmonary artery. As a result, it is difficult for the surgeon to readily determine whether the end of the jaw extends beyond the pulmonary artery, i.e., whether the pulmonary artery is entirely captured between the jaws of the end-effector.

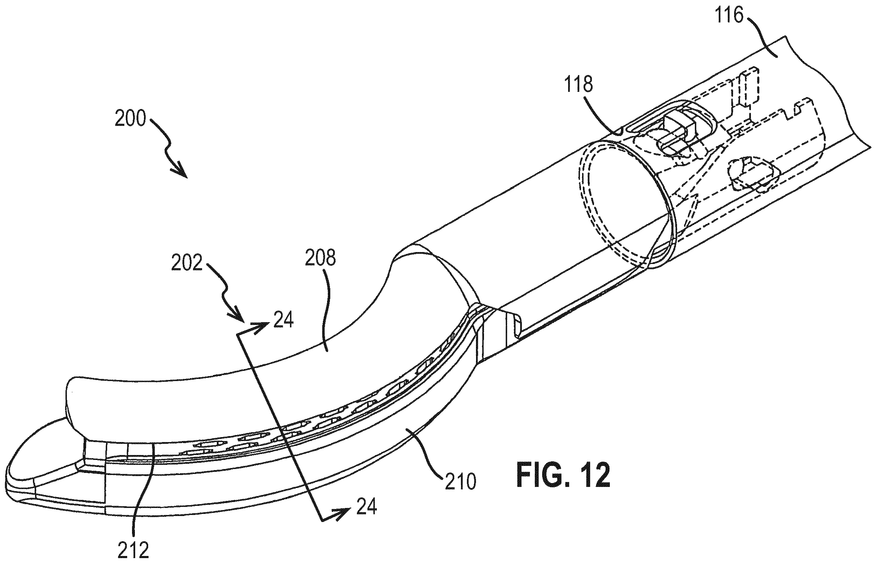

Referring to Fig. 10, the end-effector of the endocutter is curved. A curved end-effector allows a surgeon to more easily position the end-effector against the curved wall of the thoracic cavity, for example. In at least one embodiment, the curvature of the end-effector can be configured to substantially match the contour of a typical thoracic cavity wall. In these embodiments, the curvature of several thoracic cavity walls can be measured and statistically analyzed to determine the optimum profile of the curved end-effector. This profile can include several arcuate portions and, in addition, several linear portions. In other embodiments, referring to endocutter 200 of Figs. 10-14, the curvature of the thoracic cavity wall can be approximated by a single radius of curvature. Such embodiments can be simpler and less expensive to manufacture. In at least one embodiment, this radius of curvature is 1.2". In other various embodiments, the curvature of the end-effector can be configured to match the profile of the lower rectum, pelvis, or lower abdomin.

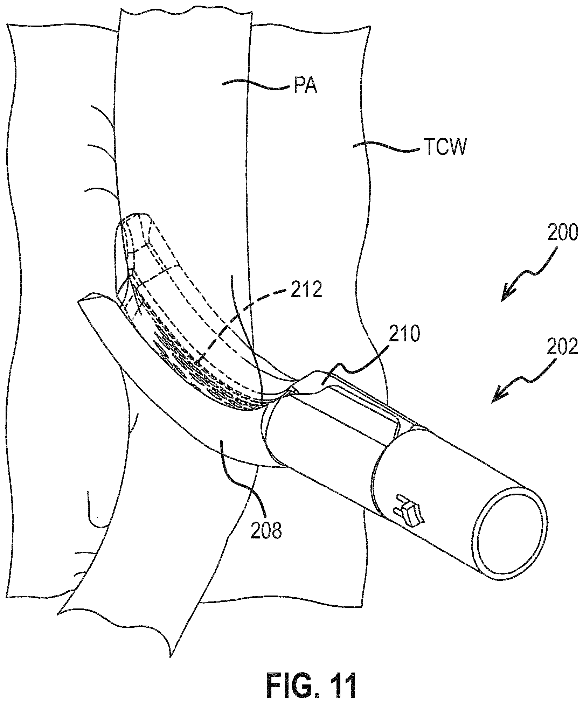

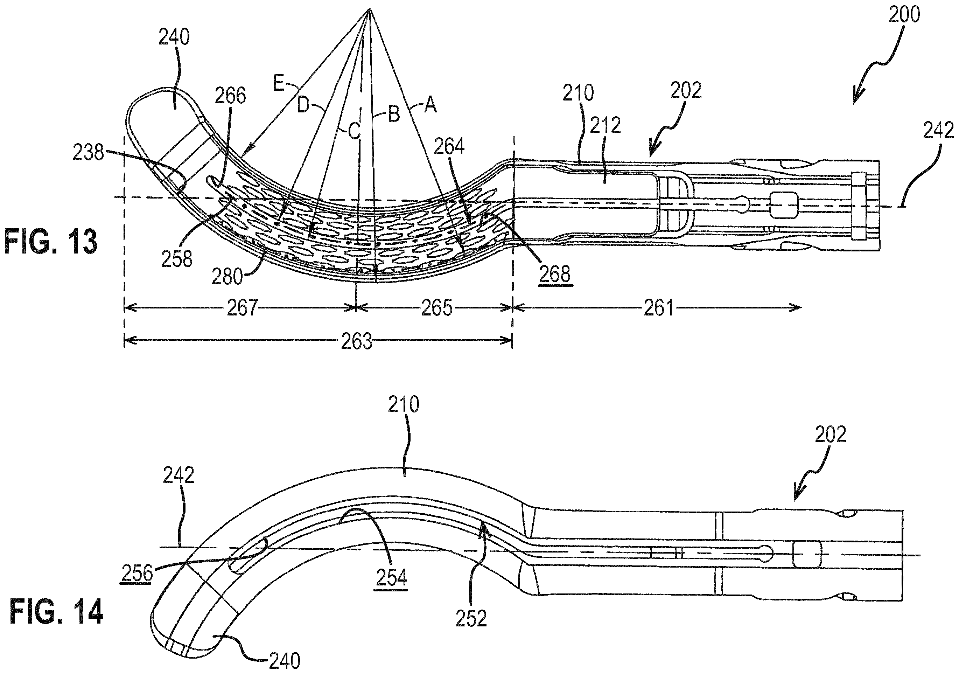

In order to transect the pulmonary artery PA, as mentioned above, a surgeon typically positions one of jaws 208 and 210 behind the pulmonary artery PA against the thoracic cavity wall TCW. Once positioned, referring to Figs. 10 and 11, closure trigger 117 is actuated to pivot jaw 208 with respect to jaw 210 such that anvil 234 contacts the pulmonary artery and compresses the pulmonary artery between anvil 234 and staple cartridge 212. Unlike previous linear end-effectors, the curved profile of end-effector 202 assists the surgeon in locating the distal end of the end-effector with respect to the pulmonary artery. More particularly, referring to Figs. 13 and 14, end 240 of jaw 210 can extend to one side of a centerline, or axis 242, defined by the distal end of shaft 106. As a result of this offset, the surgeon may be able to more readily see distal end 240 and evaluate whether the pulmonary artery is completely captured within the end-effector, for example.

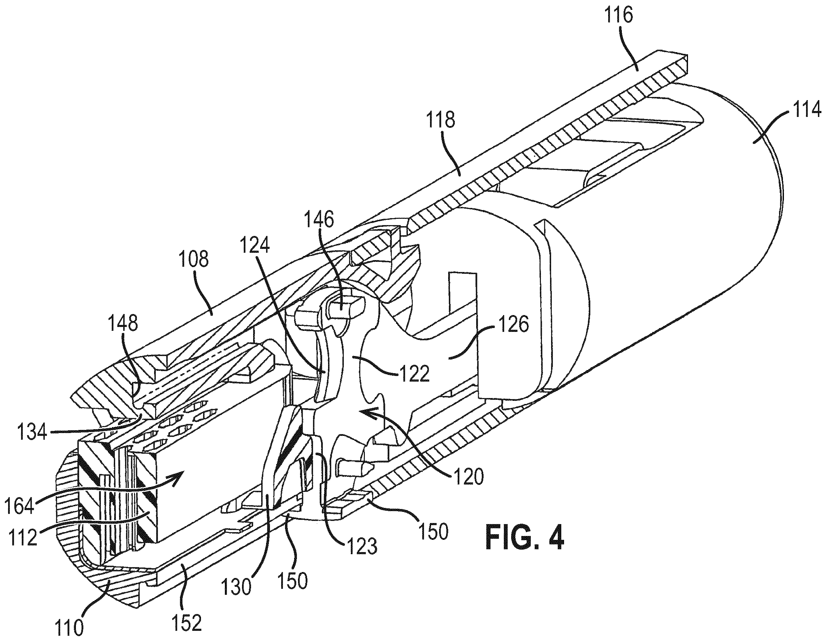

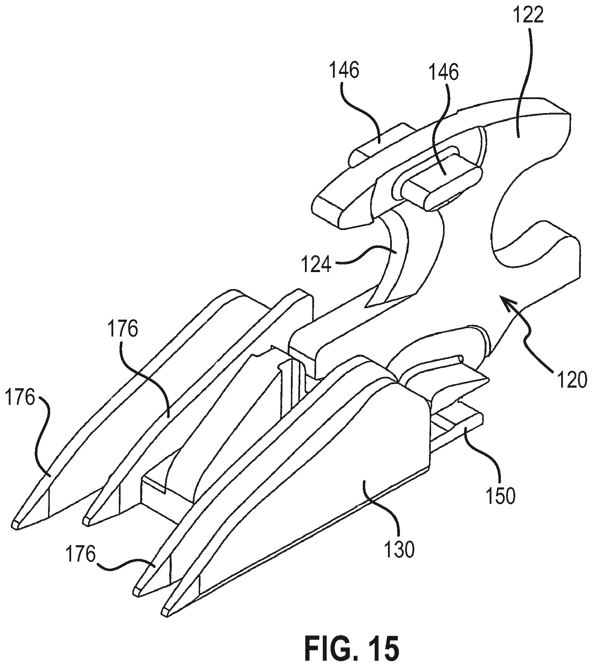

Once the jaws of the endocutter have been closed, the cutting member of the endocutter can be advanced toward the tissue, as described above. In previous endocutters, referring to Figs. 4, 15 and 16, cutting member 120 is configured to travel within linear slots defined by staple cartridge 112, staple cartridge channel 138, and anvil 134. Similarly, staple driver 130 is configured to travel within at least one linear slot defined by staple cartridge 112. As a result of these linear slots, cutting member 120 and staple driver 130 are moved in a straight line between the proximal and distal ends of the end-effector. For example, referring to Fig. 4, cutting member 120 includes first projections 146 extending from body 122 which are sized and configured to fit within slot 148 of anvil 134. Cutting member 120 further includes second projections 150 extending from body 122 which are sized and configured to retain cutting member body 122 within slot 164 of staple cartridge 112 and slot 152 of jaw 110. Accordingly, as cutting member 120 is advanced from the proximal end of the end-effector to the distal end, linear slots 148, 152 and 164 define a linear path for cutting member 120.

Referring to Figs. 13 and 14, staple cartridge 212, staple cartridge channel 238 and anvil 234 can include curved slots for controlling the movement of cutting member 120 and staple driver 130 along a curved path. These curved slots can include several arcuate portions and several linear portions. In various embodiments, the curved slots can be defined by one radius of curvature. In the embodiment illustrated in Figs. 13 and 14, staple cartridge 212 and staple cartridge channel 238 can include curved slots 264 and 252, respectively. Similar to the above, curved slots 264 and 252 can be configured to receive a portion of cutting member 120 and guide cutting member 120 along a path defined by slots 264 and 252. However, owing to the substantially linear configuration of cutting member 120, cutting member 120 may, in some circumstances, become misaligned or stuck within curved slots 264 and 252, or a corresponding curved slot in anvil 234.

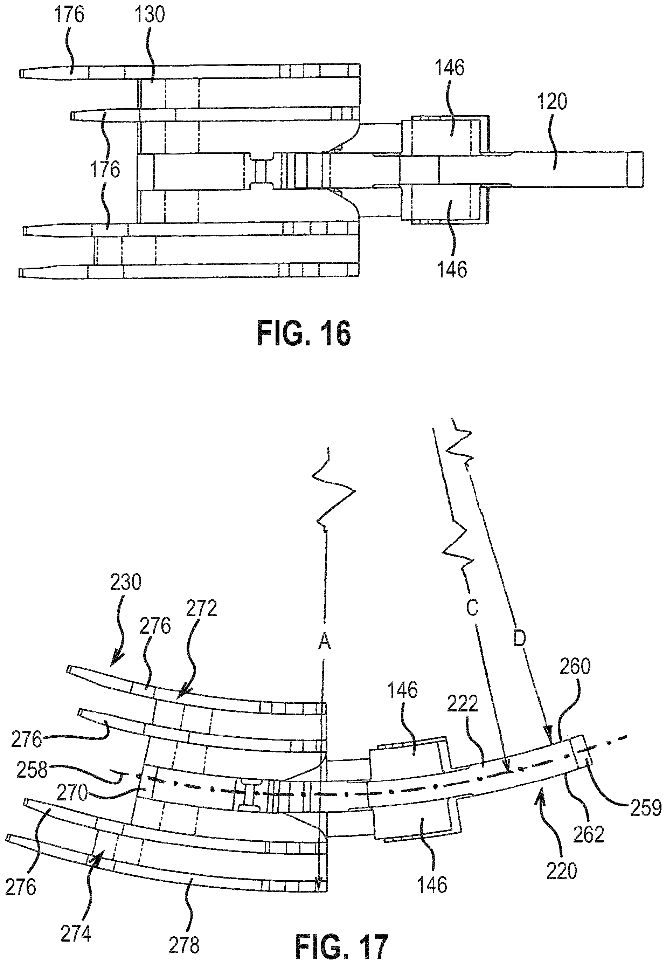

To ameliorate the above-described problem, at least a portion of the cutting member and staple driver can be curved. In at least one embodiment, the cutting member and staple driver can be configured to substantially match the curvature of the path defined by curved slots 264 and 252, i.e., path 258. More particularly, referring to Figs. 13 and 17, cutting member body 222 can include a center portion which is configured to match the radius of curvature of path 258, and a curved inner portion 260 and a curved outer portion 262 which are configured to co-operate with the sidewalls of curved slots 264 and 252. For example, curved cartridge channel slot 252 can include inner surface 254 and outer surface 256 and curved staple cartridge slot 264 can include inner surface 266 and outer surface 268 where, in the present embodiment, inner surfaces 254 and 266 are substantially defined by radius of curvature D, which is smaller than the radius of curvature of path 258, and outer surfaces 256 and 268 are substantially defined by radius of curvature C, which is larger than the radius of curvature of path 258. As illustrated in Fig. 17, inner portion 260 of cutting member 220 can be configured to closely parallel the profile of inner surfaces 254 and 266, and outer portion 262 of cutting member 220 can be configured to closely parallel the profile of outer surfaces 256 and 268. Furthermore, although not illustrated, anvil 234 can include a curved slot which, similar to slots 264 and 252, cooperates with curved cutting member 220 to guide cutting member along path 258. As a result of the above, the likelihood of cutting member 220 becoming misaligned or stuck within curved path 252 can be reduced.

Alternatively, although not illustrated, the cutting member can include slots which are configured to co-operate with features on the anvil and/or staple cartridge and guide the cutting member along a curved path. More particularly, the anvil and/or staple cartridge can each include an elongate, arcuate projection, or a plurality of projections, which define a curved, or curvilinear, path for the cutting member. The slots of the cutting member can be configured to receive the projections and guide the cutting member along the curved path. In one embodiment, one of the anvil and staple cartridge can include such a projection, or a plurality of projections, and the other of the anvil and staple cartridge can include a slot configured to receive a portion of the cutting member, as described above.

Similar to the above, at least a portion of staple driver 230 can be configured to substantially match the curvature of path 258. More particularly, referring to Fig. 17, staple driver 230 can include a center arcuate portion 270 which is configured to match the radius of curvature of path 258, and an inner arcuate portion 272 and an outer arcuate portion 274 which are configured to co-operate with the sidewalls of slots, or channels, within staple cartridge 212. Similar to staple driver 130, staple driver 230 can include ramps which are configured to lift, or deploy, staples 132 against anvil 234 positioned opposite staple cartridge 212. However, in the present embodiment, ramps 276 of staple driver 230 can be curved to deploy staples 132 along a curved staple line. More particularly, for example, the ramps can be defined by a radius of curvature which substantially matches the radius of curvature of a staple line. For example, ramp 278 is defined by a radius of curvature which substantially matches the radius of curvature of staple line 280, i.e., radius of curvature A.

Although the path of the cutting member has been described above as being defined by a single radius of curvature, the invention is not so limited. In various embodiments, referring to Figs. 13 and 14, end-effector 202 of endocutter 200 can include curved portion 263 and, in addition, linear portion 261 which is substantially collinear with an axis defined by the distal portion of shaft 116, i.e., axis 242. In at least one embodiment, curved portion 263 can further include first portion 265 and second portion 267. Referring to Fig. 13, first portion 265 can include a proximal end connected to linear portion 261 positioned along axis 242 and a distal end spaced from axis 242 wherein second portion 267 can include a proximal end connected to the distal end of first portion 265 and extend toward axis 242. Stated another way, first portion 265 can define an arcuate portion which extends away from axis 242 and second portion 267 can define an arcuate portion which extends toward axis 242. As described above, an end-effector having such a profile may facilitate the positioning of the end-effector against the wall of the thoracic cavity, for example.

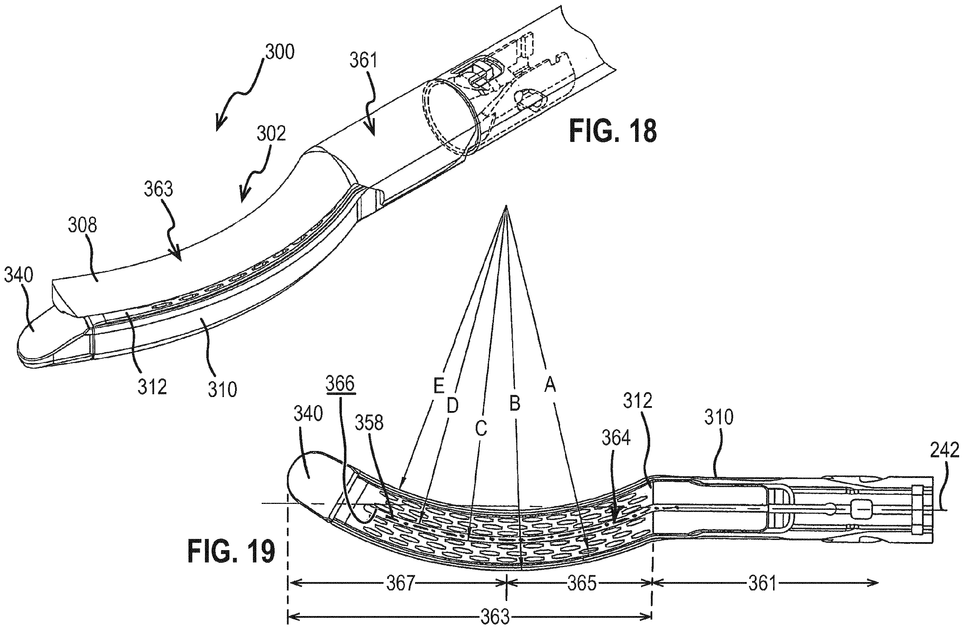

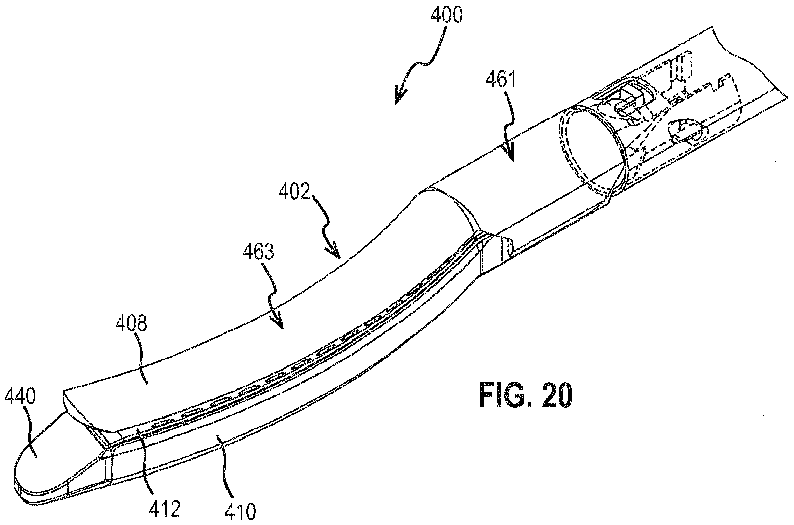

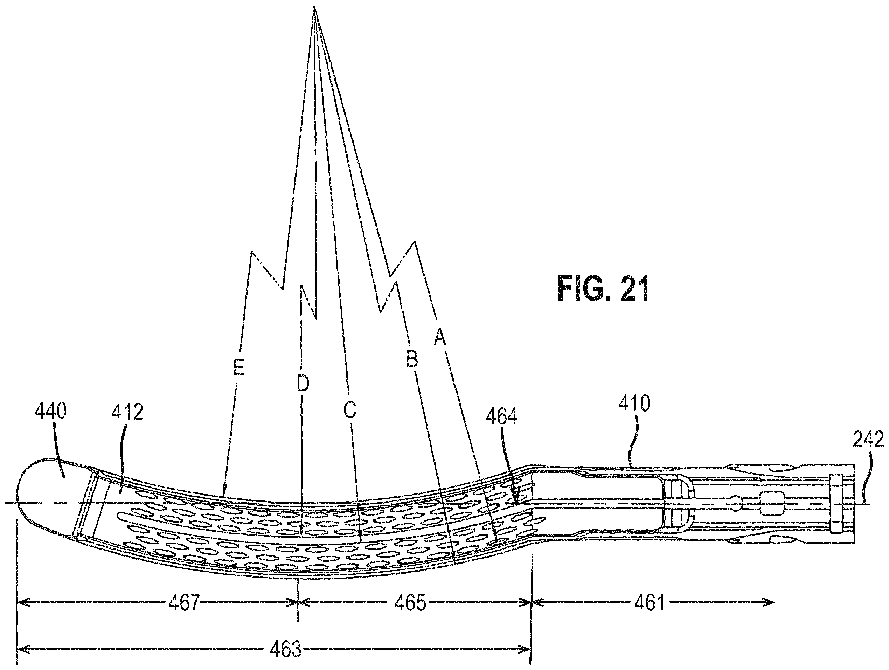

Referring to Figs. 18-21, the end-effector can include other advantageous profiles. For example, referring to Figs. 18 and 19, end-effector 302 can include linear portion 361 and curved portion 363 wherein the distal end of slot 364 can be positioned along axis 242. As a result, although the cutting member progresses along an arcuate path offset with respect to axis 242, the cutting member will stop at a point along axis 242. Thus, as long as the surgeon is able to discern the orientation of axis 242, the surgeon will know that the cutting member will not progress beyond axis 242 and can thereby gauge the point at which the tissue will no longer be transected. In another embodiment, referring to Figs. 20 and 21, end-effector 402 can include linear portion 461 and curved portion 463 wherein distal tip 440 of the end-effector lies along axis 242 although at least a portion of the end-effector is offset with respect to axis 242. In this embodiment, as long as the surgeon is able to discern the orientation of axis 242, the surgeon can gauge the location of the distal end of the end-effector when moving or dissecting tissue.

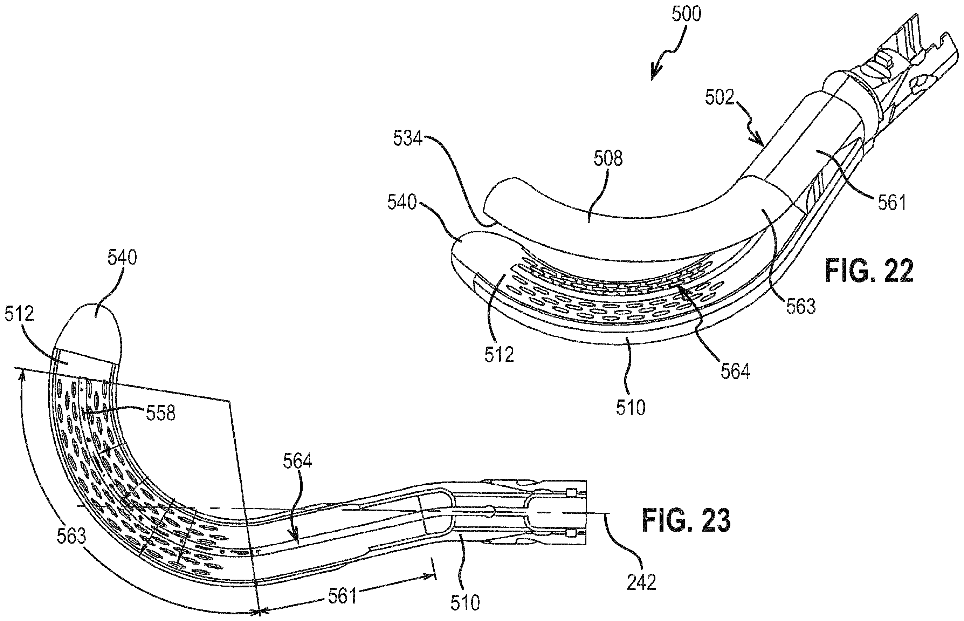

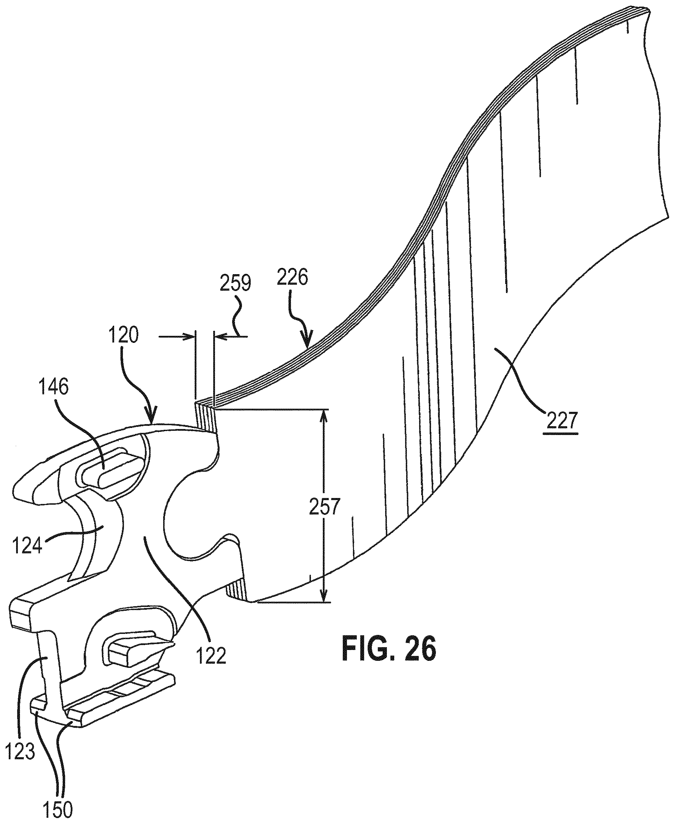

In other various embodiments, referring to Figs. 22 and 23, the end-effector can define an arcuate path for the cutting member that is defined by an angle that is greater than or equal to 90 degrees. More particularly, for example, path 558 can include linear portion 561 and curved portion 563 wherein curved portion 563 is defined by a radius of curvature that spans an arc corresponding to an approximately 110 degree angle. As a result of the significant curvature of curved portion 563, a surgeon can position a pulmonary artery, for example, entirely within curved portion 563. In various embodiments, referring to Fig. 26, staples 132 may only be positioned within cavities in curved portion 563, and not linear portion 561. In these embodiments, the staple lines can be comprised of continuous, curved rows without abrupt changes in direction within the staple line. As known in the art, abrupt changes in a staple line may provide a leak path for blood to flow therethrough. As a result of the above embodiments, the likelihood of such a leak path is reduced.

As described above, the anvil and staple cartridge can include curved slots for receiving and guiding the cutting member. In many embodiments, the anvil and the staple cartridge can be configured such that their features parallel the curved slots therein. For example, referring to Figs. 13 and 14, curved portion 263 of staple cartridge 212 can include an inner radius of curvature and an outer radius of curvature which parallel the radius of curvature of curved slot 264. More particularly, referring to Fig. 13, the inner surface of staple cartridge 212 can be defined by radius of curvature E and the outer surface of staple cartridge 212 can be defined by radius of curvature B, wherein curvatures B and E share a substantially common radial point with radius of curvatures C and D which, as described above, substantially define the inner and outer surfaces of slot 264. However, in various embodiments, although not illustrated, the inner and outer surfaces of the anvil and/or staple cartridge, or any other features thereof, may be non-parallel to the curved slot. In these embodiments, the anvil and staple cartridge, and the jaws surrounding them, may be configured to achieve any suitable configuration or purpose.

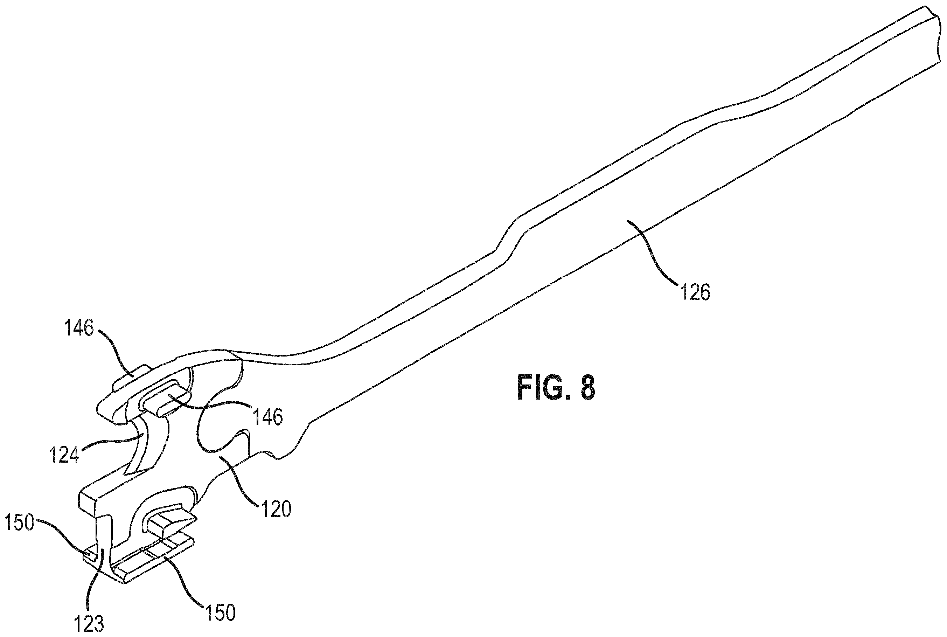

In previous endocutters, as described above and referring to Figs. 4 and 8, linear drive bar 126 is configured to advance cutting member 120 along a linear path and, as a result, drive bar 126 is constructed such that is rigid and does not substantially deflect. After cutting member 120 has been advanced into slots 148, 164 and 152 of anvil 134, staple cartridge 112, and staple cartridge channel 138, respectively, at least a portion of drive bar 126 can enter into slots 148, 164 and 152. However, although cutting member 120 is guided and supported within slots 148, 164, and 152, drive bar 126, in these previous devices, is unsupported within slots 148, 164, and 152. As a result, drive bar 126 may deflect or buckle in an uncontrollable and undesirable manner when load is transmitted therethrough.

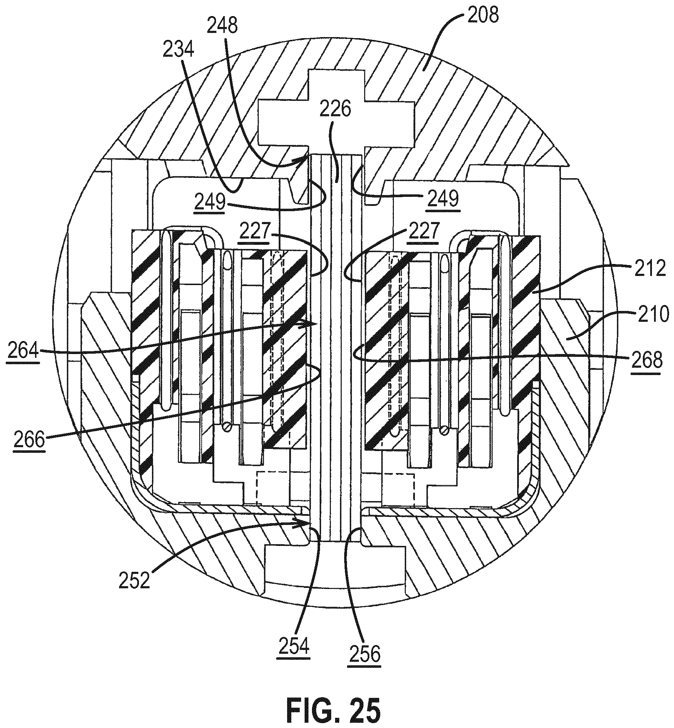

In various embodiments, a flexible drive bar can be used to advance the cutting member within the end-effector. More particularly, in order for the drive bar to be advanced into and translate within the curved slots of the end-effector, the drive bar can deflect to closely parallel the curvature of the curved slots of the end-effector. In various embodiments, unlike previous endocutters, the slots within the anvil and staple cartridge can be configured to support the flexible driver bar. More particularly, after cutting member 120 has been at least partially advanced within slots 248, 264, and 252, referring to Fig. 25, at least a portion of drive bar 226 can enter slots 248, 264, and 252. Slot 248 can include support surfaces 249 which are configured to abut, or be positioned closely adjacent to, side surfaces 227 of drive bar 226. Similarly, surfaces 254 and 256 of slot 252 and surfaces 266 and 268 of slot 264 can also support the drive bar. While these features are particularly advantageous when used with curved end-effectors, they can also be used in linear end-effectors. In these embodiments, even though the slots may be linear, the slots can support the driver, whether rigid or flexible, and prevent it from buckling in the event that it is overloaded, for example.

Although flexible drive bar 226 can be used to advance linear cutting member 120 and linear staple driver 130 within a curved end-effector, as described above, flexible drive bar 226 can also be used to advance curved cutting members and staple drivers, such as cutting member 220 and staple driver 230, for example, within a curved end-effector. Furthermore, although not illustrated, one of the anvil and staple cartridge can include a slot configured to receive and guide the cutting member and the other of the anvil and staple cartridge can include a slot configured to receive and support the drive bar. In these embodiments, the slot which is configured to receive the cutting member can have a different geometry than the slot which is configured to receive the drive bar. Accordingly, the cutting member and the drive bar can have different thicknesses, for example.

In various embodiments, the support surfaces of slots 248, 264 and 252 may be continuous, i.e., they may be configured to contact drive bar 226 continuously along the length thereof, or, alternatively, slots 248, 264 and 252 may be configured to contact drive bar 226 at various, spaced-apart locations. In these embodiments, projections may extend from the slot walls to define the path of the cutting member and the drive bar. In various embodiments, drive bar 226 may be comprised of a flexible, unitary material such as plastic, for example. Alternatively, referring to Figs. 25 and 26, drive bar 226 may be comprised of a laminated material, i.e., a material comprised of two or more materials bonded together. In these embodiments, two or more strips of material may be glued together where the strips have the same cross-sectional geometry, or, alternatively, different cross-sectional geometries. Furthermore, the strips may be comprised of the same material or different materials. The cross-sectional geometries and materials of the above-described embodiments may be selected such that the drive bar is more flexible when deflected in one direction and less flexible when deflected in a different direction.

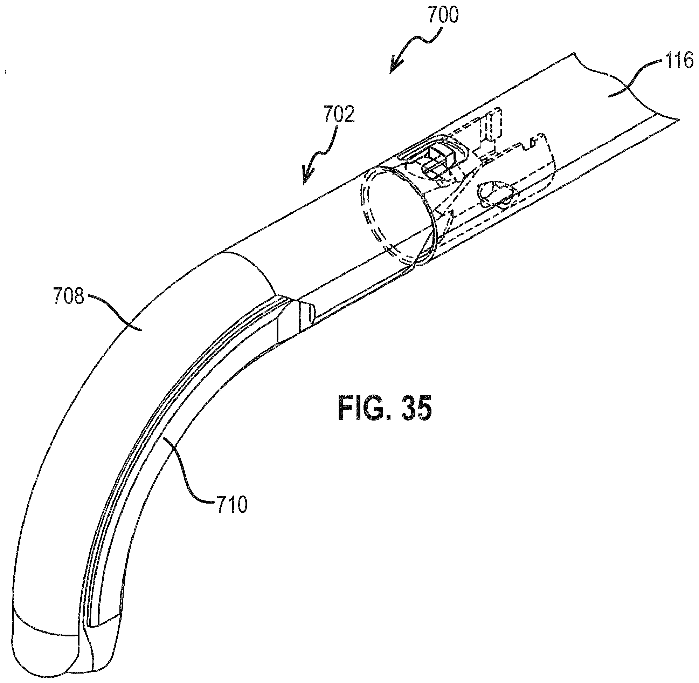

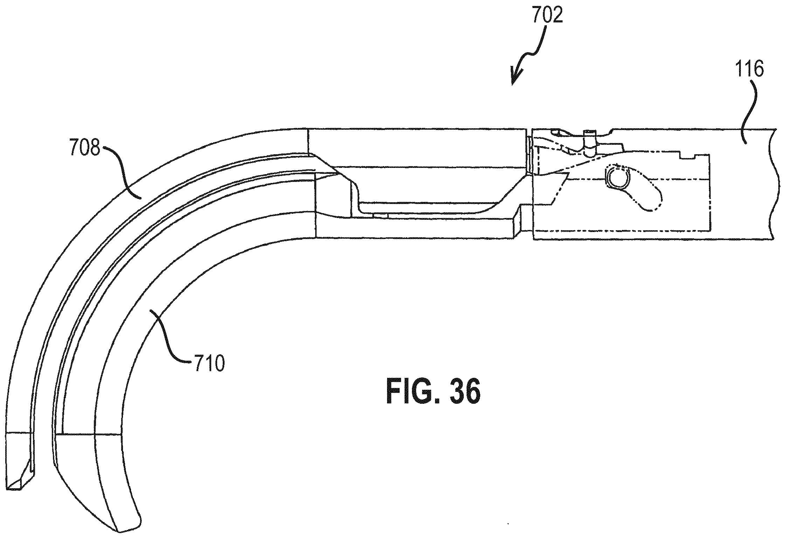

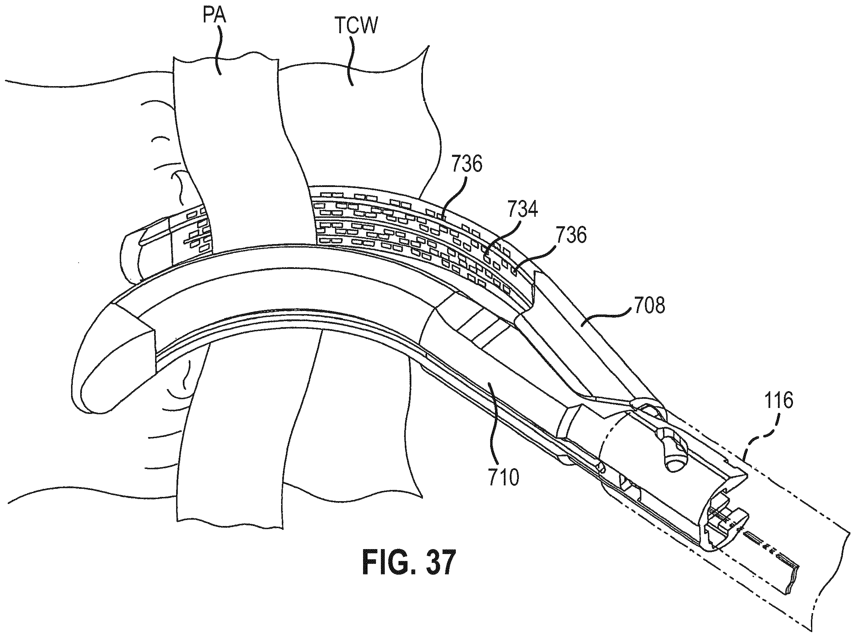

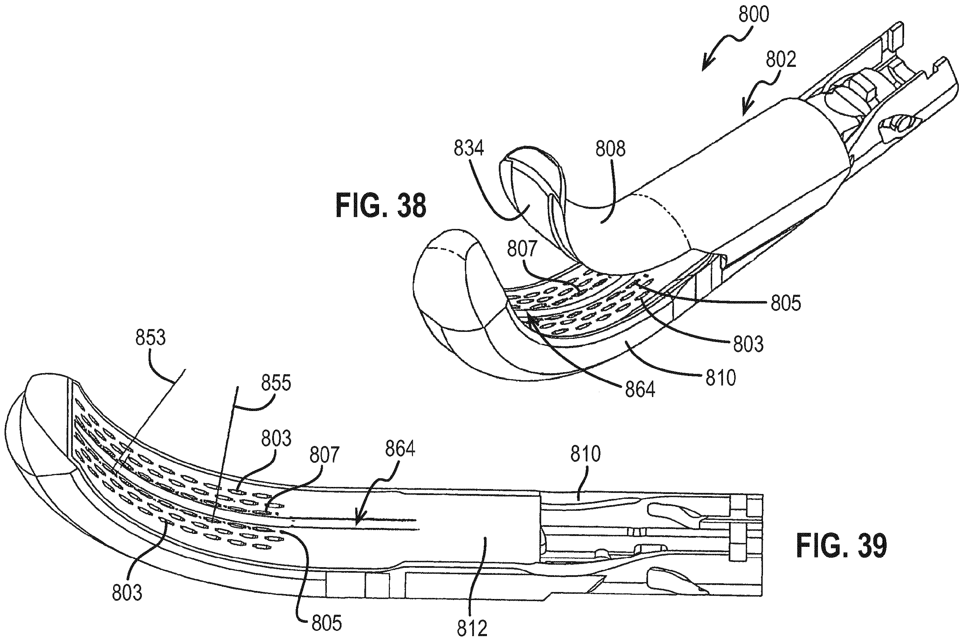

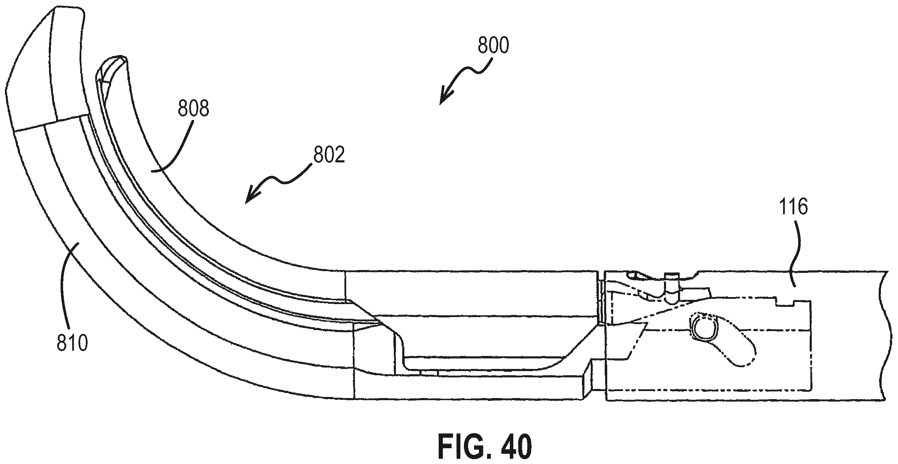

As described above, the curvature of an end-effector can be selected such that it facilitates the placement of the end-effector in a particular surgical site. In various embodiments, referring to Figs. 35-37 and 38-40, the end-effector can be curved in a downward or upward direction, i.e., it can be curved in a plane that is substantially parallel to planes defined by the staple lines. More particularly, referring to Figs. 38 and 39, staple cavities 803, which are configured to store staples 132 therein, are positioned along staple lines 805 and 807, for example, such that staples 132, when they are deployed from staple cartridge 812, are deployed in substantially parallel planes which are at least partially defined by staple lines 805 and 807.

For each parallel plane described above, as a result of these upward and/or downward curvatures, staples 132 can be deployed along axes which are co-planar, but not parallel. More particularly, referring to Fig. 39, a first staple 132 (not illustrated in Fig. 39) can be deployed from its staple cavity 803 along axis 853 and a second staple 132 can be deployed from its staple cavity 803 along axis 855. While axis 853 and axis 855 can be co-planar, as illustrated in Fig. 39, axis 853 and axis 855 are not parallel. In some embodiments, the axes defined by staple cavities 803 can converge, as illustrated in Figs. 38 and 39, or diverge, as illustrated in Figs. 35-37. In various embodiments, the staple deployment axes can define an angle therebetween which is greater than or equal to 30 degrees. In other various embodiments, the axes can be substantially perpendicular and, in further embodiments, the axes can define an angle that is greater than ninety degrees.

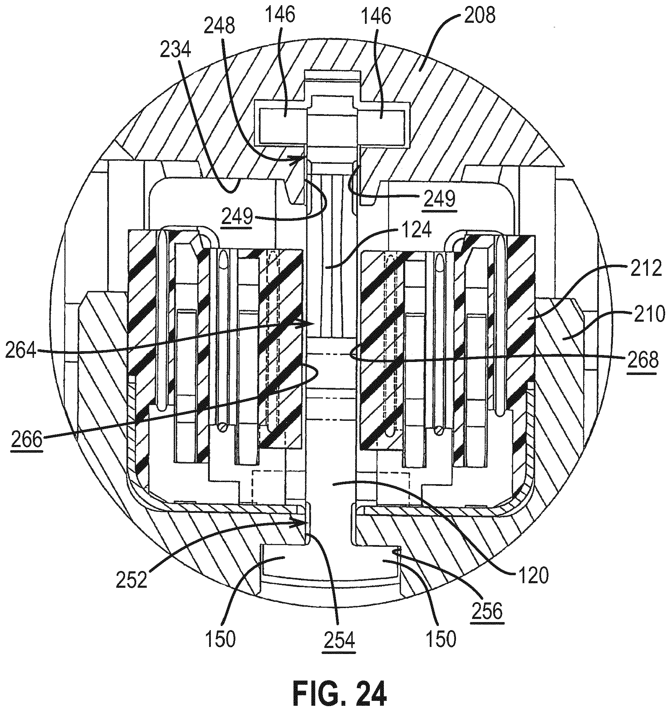

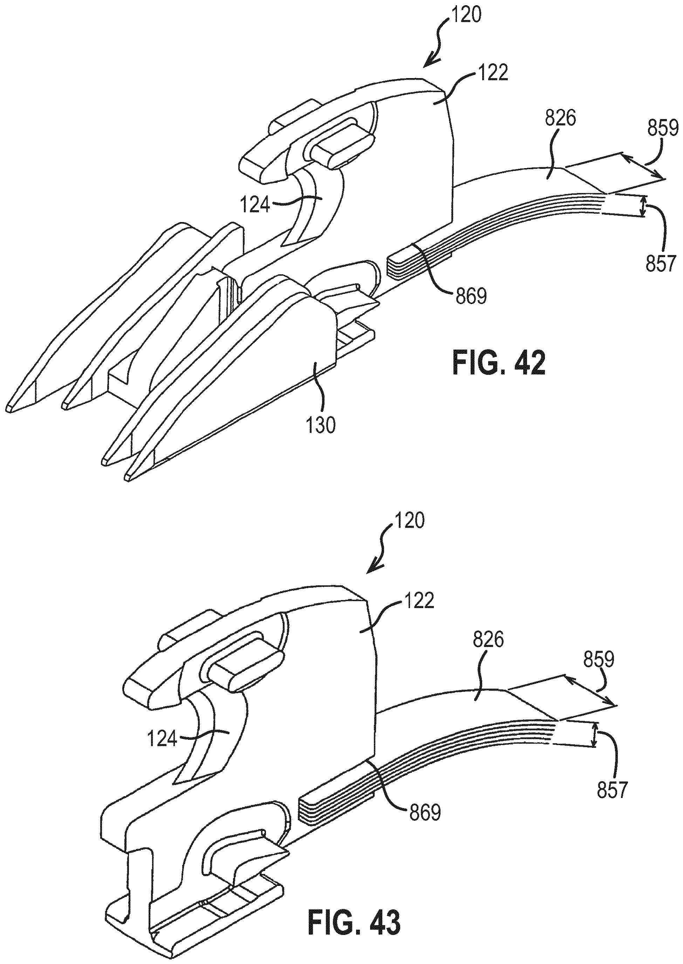

As described above, an endocutter can include a cutting member which is advanced through and guided by curved slots in the staple cartridge and/or anvil. For example, referring to Figs. 38-43, staple cartridge 812 can include slot 864 which is configured to receive and guide cutting member 120. Similar to the above, endocutter 800 can further include a drive bar for advancing cutting member 120 within slot 864 of staple cartridge 812, however, owing to the direction and degree of the curvature of staple cartridge 812, some drive bars may be largely unsuitable for use with endocutter 700 or 800, for example. More particularly, the illustrated drive bars 126 and 226 in Figs. 4 and 24, respectively, owing to their cross-sectional geometries, may not be particularly well-suited to flex in a substantially downward or substantially upward direction as required by endocutters 700 and 800, respectively. Referring to Fig. 26, for example, the illustrated cross-section of drive bar 226 is substantially rectangular and is defined by height 257 and width 259. As illustrated in Fig. 26, height 257 is substantially greater than width 259 and, as a result, the cross-section of the illustrated drive bar 226 has a moment of inertia with respect to height 257 that is substantially greater than the moment of inertia with respect to width 259. Accordingly, the illustrated drive bar 226 is substantially less flexible with respect to height 257 than width 259 and may not be able to sufficiently bend in the substantially downward and upward directions described above. It is important to note that drive bars 126 and 226 are not limited to the configurations described above. On the contrary, drive bars 126 and 226 can have cross-sections in which the width is greater than the height. Any reference in this paragraph to drive bars 126 and 226 are references to the particular drive bars 126 and 226 that happen to be illustrated in Figs. 4 and 24, respectively.

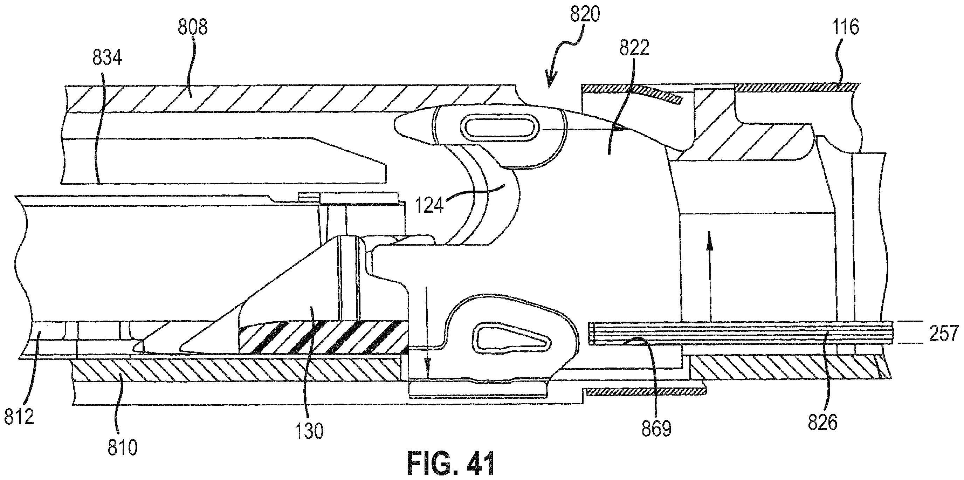

Referring to Figs. 41-43, endocutter 800 can include drive bar 826 which, similar to drive bar 226, is configured to advance cutting member 120, or a curved cutting member, through curved slots in an end-effector. In various embodiments, drive bar 826 can include a cross-sectional geometry having a width 859 that is greater than its height 857. In these embodiments, the moment of inertia of the cross-section with respect to height 857 is less than the moment of inertia with respect to width 859. As a result, drive bar 826 can be more flexible with respect to height 857, i.e., in the upward and downward directions, than with respect to width 859. In at least one embodiment, width 859 can be approximately .12" and height 857 can be approximately .05". Although drive bar 826 is illustrated as having a rectangular cross-section, the invention is not so limited. On the contrary, the cross-section of drive bar 826 can include various embodiments in which the width of the drive bar cross-section is greater than its height. In at least one embodiment, drive bar 826 can include a cross-section defined by a width and a height wherein the width is greater than the height, and wherein the width defines an axis that is not parallel to an axis defined by cutting edge 124 of cutting member 120. In various embodiments, as known in the art, cutting edge 124 can include a knife edge or a wire configured to conduct current therethrough. Furthermore, in various embodiments, the drive bar can be asymmetric with respect to centerline 224 of the distal end of shaft 116, for example. In these embodiments, as a result, drive bar 826 can be predisposed to bending in a pre-determined direction.

Similar to drive bar 226, drive bar 826 can be comprised of one material or, alternatively, several layers of material bonded together. As above, the flexibility of drive bar 826 can be pre-determined by the types of materials used and the arrangement of the layers within the drive bar. Referring to Fig. 41, cutting member body 822 can include slot 869 which is configured to receive the distal end of drive bar 826. In the present embodiment, slot 869 is configured to receive drive bar 826 in a press-fit relationship, however, other means, such as adhesive or fasteners, can be used to secure drive bar 826 to cutting member 820. Similar to the above, staple cartridge 812 can include a slot configured to receive and support drive bar 826 when it enters into staple cartridge 812. In various embodiments, although not illustrated, anvil 834 could be configured to receive and support drive bar 826.

As described above, the jaws of an endocutter can be placed on opposite sides of several layers of tissue, for example, and then closed onto the tissue. In the illustrated embodiments, referring to Fig. 4, jaw 108 can be pivoted between opened and closed positions with respect to jaw 110 via the interaction of inner portion 114 and outer sleeve 116 of shaft 106 in a known manner. Although not illustrated, jaw 108 is connected to jaw 110 via a pivot connection such that when inner portion 114 moves jaw 108 relative to outer sleeve 116, jaw 108 is pivoted toward jaw 110. Throughout the movement of jaw 108, the proximal portion of jaw 108, i.e., proximal portion 111, is positioned closer to jaw 110 than its distal portion, i.e., distal portion 113, until jaw 108 is brought into its final position opposite staple cartridge 112. In this final, closed position, distal portion 113 and proximal portion 111 can be substantially equidistant from staple cartridge 112. However, as a result of distal portion 113 being the last portion of jaw 108 to reach its final position, a portion of the tissue, or an artery, for example, can escape from between jaws 108 and 110 before distal portion 113 is moved into its final position. Accordingly, the surgeon may have to reopen the jaws and reposition the end-effector in an attempt to properly capture the tissue, or artery, therebetween.

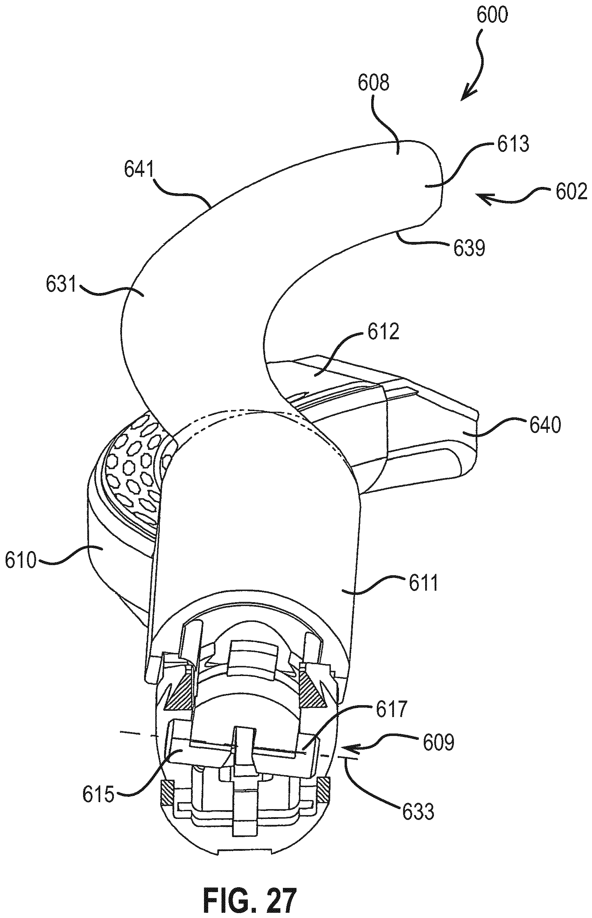

As detailed below, an end-effector can be configured to capture the tissue, or an artery, between the distal and proximal portions of the end-effector before the jaws are moved into their final position. In at least one embodiment, referring to Figs. 27-34, jaw 608 can be pivotally connected to jaw 610 via pivot connection 609. Pivot connection 609 can include first trunnion 615 and second trunnion 617 extending from jaw 608, and, in addition, first slot 619 and second slot 621 in jaw 610. Trunnions 615 and 617 can be sized and configured to fit within slots 619 and 621, respectively, such that pivot connection 609 allows for relative rotational and translation movement between jaw 608 and jaw 610. In other alternative embodiments, jaw 608 may include slots 619 and 621 and jaw 610 may include trunnions 615 and 617, or any other combination thereof.

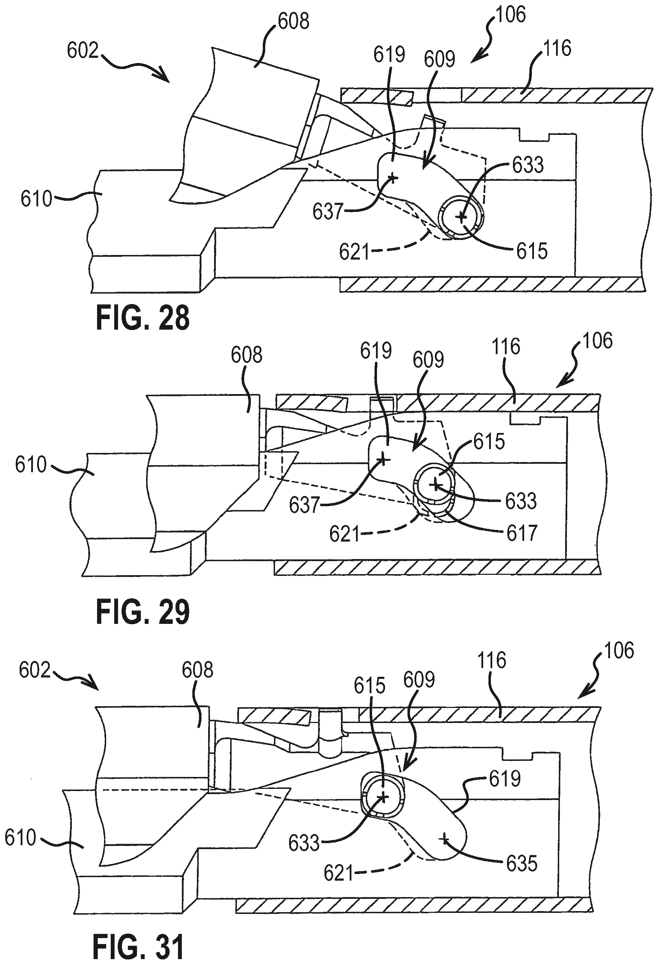

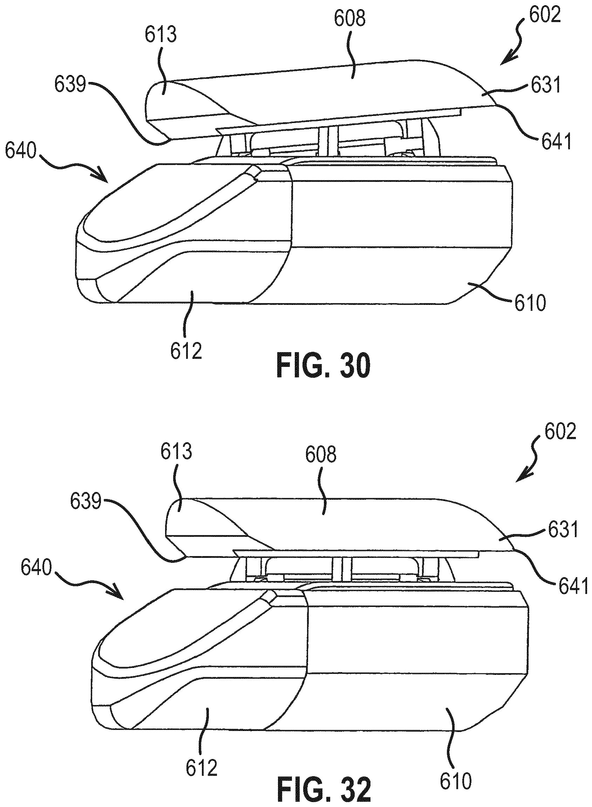

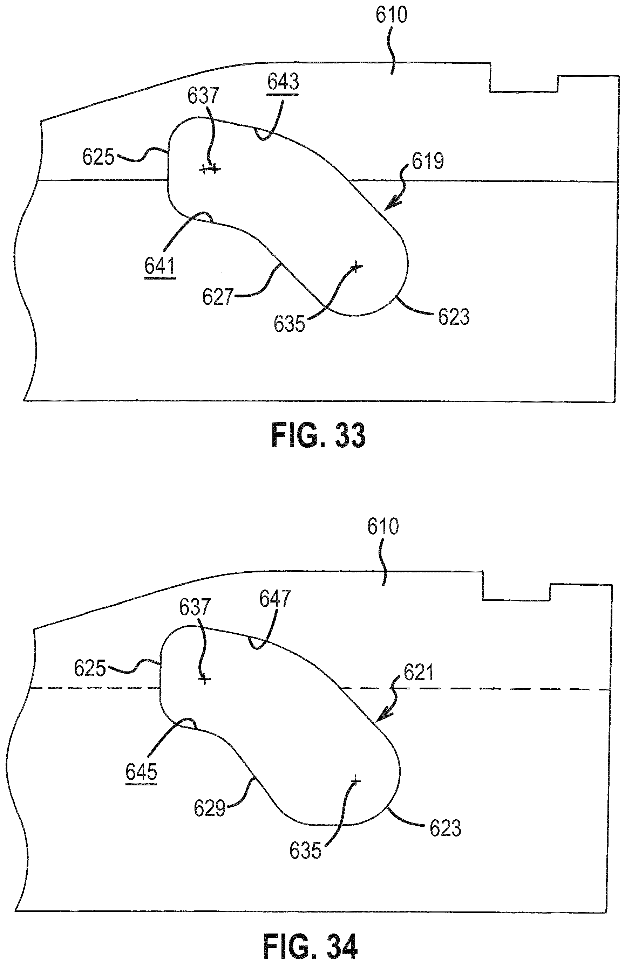

Referring to Figs. 28, 29 and 31 which schematically illustrate slot 619 in solid and slot 621 in dashes, trunnions 615 and 617 are configured to travel within slots 619 and 621, respectively, and define the relative movement between jaws 608 and 610. In the present embodiment, slots 619 and 621 define two different arcuate paths for trunnions 615 and 617. More particularly, referring to Figs. 33 and 34, slot 619 includes first portion 623, second portion 625, and intermediate portion 627 extending therebetween wherein slot 621 also includes first portion 623 and second portion 625, however, slot 621 includes an intermediate portion, i.e., portion 629, which is different than intermediate portion 627. Referring to Fig. 27, as a result of slots 619 and 621 having different intermediate portions, slots 619 and 621 can cause jaw 608 to tilt, or otherwise move in a non-symmetrical manner, with respect to jaw 610 as it is opened and closed. Advantageously, referring to Figs. 30 and 32, such an asymmetric motion, or tilting, can allow distal portion 613 of jaw 608 to be placed in close proximity to staple cartridge 612 before the intermediate portion of jaw 608, i.e., portion 631, is moved into its final position illustrated in Fig. 32. As a result, referring to Fig. 30, an end-effector in accordance with the above can be used to capture tissue, or an artery, between proximal end 611 and distal end 613 before intermediate portion 631 is moved into its final, or closed, position. As a result, the possibility of a portion of the tissue, or artery, escaping from between jaws 608 and 610 is reduced. In addition to the above, the distal ends of jaws 608 and 610 can be brought into close opposition to each other in order to grip delicate tissue, for example, without having to completely close the end-effector.

As outlined above, slots 619 and 621 can define different paths for trunnions 615 and 617, respectively, when jaw 608 is moved between an open and a closed position. When jaw 608 is in its open position, referring to Fig. 28, trunnions 615 and 617 are positioned within first portions 623 of slots 619 and 621. In this position, axis 633, which is defined by trunnions 615 and 617, is substantially collinear with axis 635 defined between first portions 623 of slots 619 and 621. Thereafter, jaw 608 can be moved distally such that trunnions 615 and 617 move upward through slots 619 and 621. Owing to the asymmetric configurations of slots 619 and 621, referring to Fig. 27 which illustrates jaw 108 in a partially closed position, trunnion 615 is elevated to a relatively higher position with respect to trunnion 617, as evidenced by the tilting of axis 633. In this position, an inner edge of jaw 608, i.e., edge 639, can be in closer proximity to staple cartridge 612 than an outer edge of jaw 608, i.e., edge 641. Advantageously, as a result, inner edge 639 can be brought into contact against the tissue, or an artery, for example, allowing the surgeon to evaluate the position of the end-effector with respect to the tissue, or artery, without having to bring the entire anvil 634 of jaw 608 against the tissue. This feature may be particularly advantageous when the end-effector is positioned around a pulmonary artery as pulmonary arteries are especially susceptible to rupture.

After the tissue, or artery, has been captured between the proximal and distal ends of the end-effector, referring to Figs. 31 and 32, jaw 608 can be moved into its final, or closed, position with respect to staple cartridge 612. In this position, axis 633, which is defined by trunnions 615 and 617, can be substantially collinear with axis 637 defined between second portions 625 of slots 619 and 621. Furthermore, in this final position, intermediate portion 631, distal portion 613 and proximal portion 611 can be equidistant from staple cartridge 612. Similarly, outer edge 641 and inner edge 639 can also be positioned equidistant with respect to staple cartridge 612. In this final position, tissue, or an artery, for example, can be securely retained between jaws 608 and 610. The above features can be utilized with a linear end-effector, for example, to achieve the advantages described above.

In various embodiments, slots 619 and 621 can define paths having different centerlines wherein each centerline can be defined as the line equidistant from the top and bottom surfaces of each slot. For example, referring to Figs. 33 and 34, slot 619 can include bottom surface 643 and top surface 645 which define a centerline therebetween that is different than the centerline defined by bottom surface 647 and top surface 651 of slot 621. In these embodiments, slots 619 and 621 can be configured to closely retain trunnions 615 and 617 between these top and bottom surfaces such that axis 633 of trunnions 615 and 617 substantially travels along the centerlines of slots 619 and 621. In various embodiments, jaws 608 and 610 can be configured such that trunnions 615 and 617 contact bottom surfaces 641 and 647 of slots 619 and 621. In these embodiments, jaw 608 can be biased by a spring, for example, such that trunnions 615 and 617 are positioned against bottom surfaces 641 and 647 throughout the movement of jaw 608. Owing to different profiles for bottom surfaces 641 and 647, the advantages described above can be achieved.

As described above, once the jaws of the end-effector are closed onto the layers of tissue, for example, staples can be deployed into the tissue. However, oftentimes, the layers of tissue are very thin and the staples may not properly capture the tissue therein. To ameliorate this problem, as known in the art, buttress material can be placed on one or both sides of the tissue to support the tissue as it is being stapled. In such embodiments, the purchase of the staples is improved and the clamping force of the staples may be spread more evenly across the buttress material. In various embodiments, the buttress material can be comprised of a bioabsorbable material such that it can dissolve away during the healing process. Previously, however, the buttress material has been provided in linear strips which are configured to accommodate linear staple lines and end-effectors. Such linear strips may be unsuitable for use with endocutters having a curved end-effector configured to deploy staples in curved staple lines.

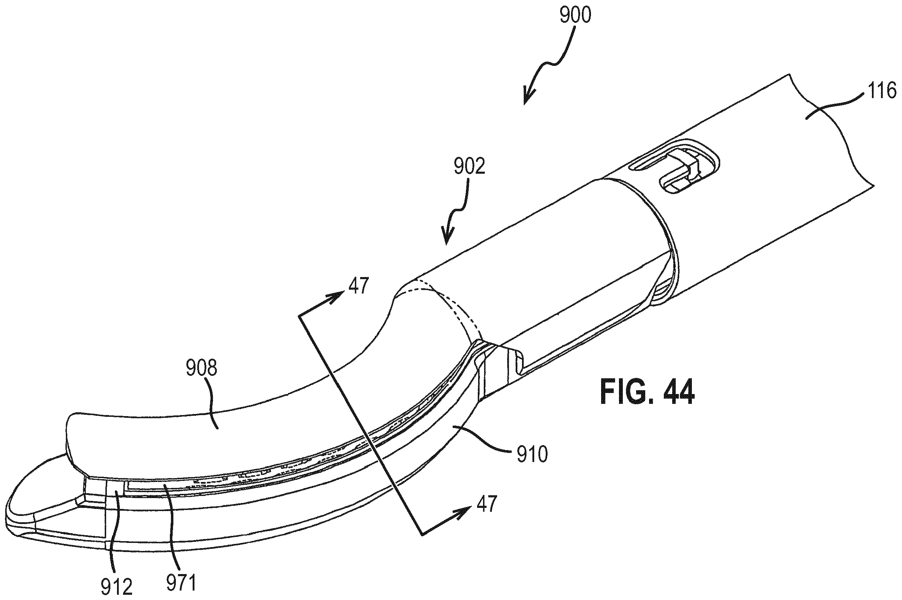

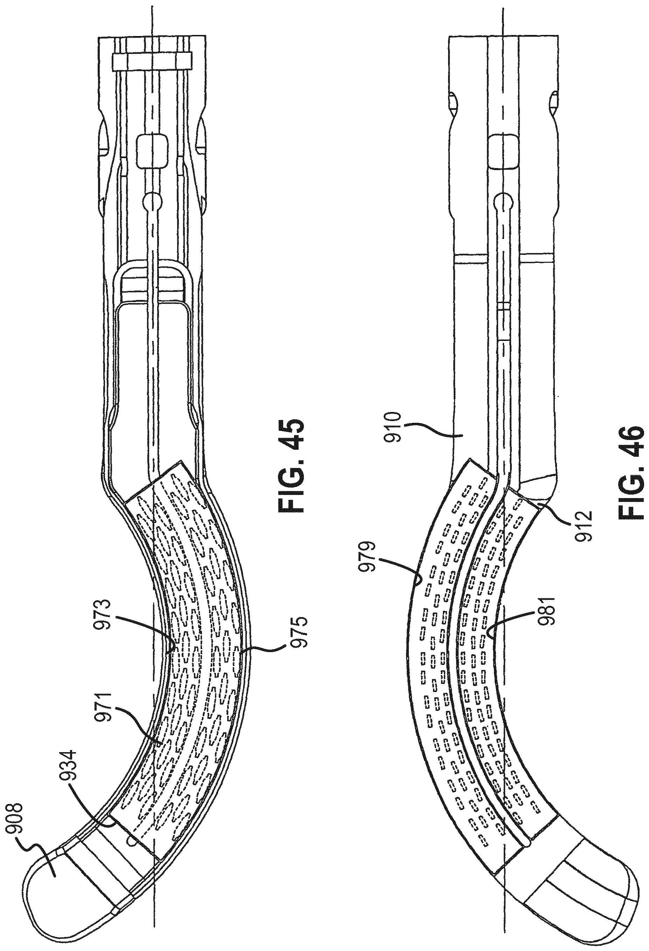

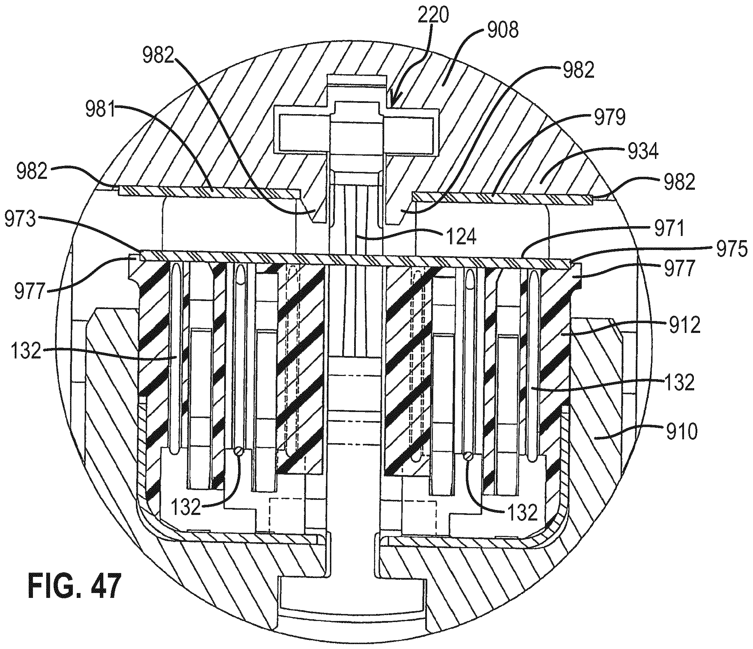

In accordance with an embodiment of the present invention, referring to Figs. 44-47, curved staple cartridge 912 is configured to receive a curved piece, or pieces, of buttress material thereon, such as buttress material 971. Curved buttress material 971 can include inner edge 973 which can be configured to substantially parallel the inner radius of curvature of jaw 910, and, in addition, outer edge 975 which can be configured to substantially parallel the outer radius of curvature of jaw 910. referring to Fig. 47, staple cartridge 912 includes lip 977 extending therefrom which is configured to retain buttress material 971 on staple cartridge 912. More particularly, lip 977, as illustrated, is configured to limit lateral movement of buttress material 971 with respect to staple cartridge 912 and, although not illustrated, lip 977 is configured to extend distal to and proximal to the ends of the buttress material to limit relative axial movement between buttress material 977 and staple cartridge 912. Similar to the above, curved anvil 934 can be configured to receive a piece, or pieces, of curved buttress material thereon, such as buttress material 979 and 981, for example. Referring to Fig. 47, anvil 934 can include several lips 982 which are configured to limit relative movement between buttress material 979 and 981 and anvil 934. In various embodiments, an adhesive, such as cyanoacrilate, for example, can be applied to the buttress material, anvil and/or staple cartridge to further limit the movement of the buttress material or otherwise prevent the mobilization thereof.

As a result of the above, a surgeon may be able to position the end-effector into a surgical site without the buttress material falling off or moving relative to the staple cartridge and/or anvil. Once positioned, cutting member 120 can be advanced to cut buttress material 971. More specifically, referring to Fig. 47, cutting edge 924 can be aligned with buttress material 971 such that it cuts the buttress material as cutting member 920 is advanced through staple cartridge 912. However, in some circumstances, the cutting member may at least partially dislodge the buttress material relative to the staple cartridge. This relative movement may especially occur when the buttress material is thick, or, the cutting member must cut more than one piece of buttress material at a time. To ameliorate this problem, the buttress material may include a series of perforations, for example, positioned along the path in which the cutting member will cut the buttress material. In these embodiments, these perforations may be formed along a radius of curvature which is parallel to and positioned intermediate two curved staple rows. In other various embodiments, the buttress material may include other features which disrupt the cross-sectional thickness of the buttress material to facilitate the cutting of the buttress material. As a result of the above, less force may be required to cut the buttress material and, accordingly, it is less likely the buttress material may slide, for example, when it is cut.

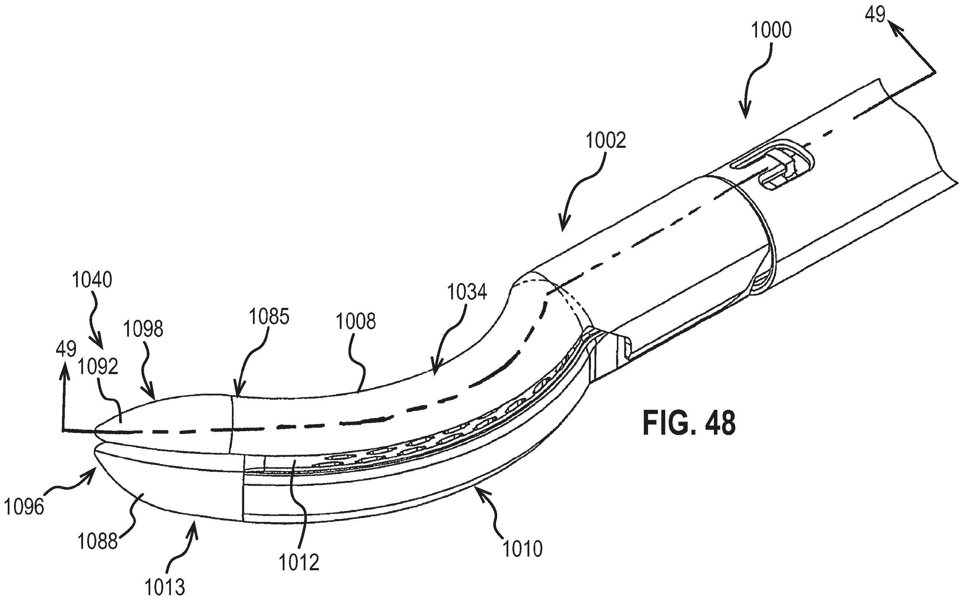

Figs. 48-50 illustrate another surgical instrument. As can be seen in these Figures, the surgical instrument 1000 includes an end-effector 1002 that has a first jaw 1008 and a second jaw 1010. The second jaw 1010 may comprise a channel 1038 that is configured to operably support a staple cartridge 1012 therein. Staple cartridge 1012 may be removably supported in the channel 1038 or, in various embodiments, staple cartridge 1012 may form an integral part of the second jaw 1010. The surgical instrument 1000 further includes a movable anvil 1034 that may be movably coupled to the lower jaw 1010 in the various manners described above or in other manners that are known in the art.

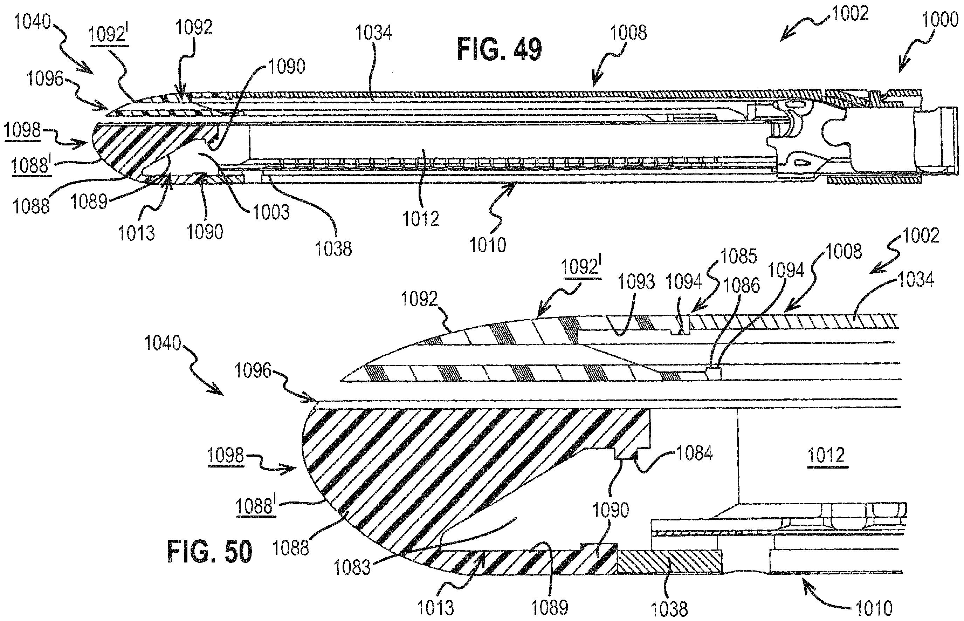

In the embodiment depicted in Figs. 48-50, the end effector 1002 has a distal end generally designated as 1040. As can further be seen in those Figures, the staple cartridge 1012 has a blunt first tip portion 1088 thereon. The first tip portion 1088 may be integrally formed (molded, machined, etc.) on the distal end 1013 of the staple cartridge 1012 or it may comprise a separate piece that may be formed with a cavity 1089 (Fig. 50) configured to receive a nose 1083 of a conventional staple cartridge 1012. The first tip portion 1088 can include snap features 1090 (Fig. 50) or other suitable retainer portions formed therein to retainingly mate with complementary retention grooves 1084 formed in the nose 1083. In addition, or in the alternative, the first tip portion 1088 may be affixed to the cartridge 1012 by adhesive such as, for example, cyanoacrylates, light-curable acrylics, polyurethanes, silicones, epoxies, and ultraviolet curable adhesives such as Henkel Loctite ®. In other embodiments, a combination of snap features and grooves may be provided in both the staple cartridge 1012 and the first tip portion 1088. Still other forms of fasteners and fastener arrangements may be used to affix the first tip portion 1088 to the staple cartridge 1012. In other embodiments, the first tip portion 1088 may be affixed to the channel 1038. As can be seen in Fig. 50, the first tip portion 1088 has a first upwardly extending curved outer surface.

Similarly, in this embodiment, the anvil 1034 may be equipped with a second tip portion 1092. The second tip portion 1092 may be integrally formed (molded, machined, etc.) on the distal end 1085 of the anvil 1034 or it may comprise a separate piece that may be formed with a cavity 1093 configured to receive an end portion of a conventional anvil 1034 with snap features 1094 or other suitable retainer portions formed therein to retainingly mate with complementary retention grooves 1086 formed in distal end 1085. In addition, or in the alternative, the second tip portion 1092 may be affixed to the anvil 1034 by adhesive such as, for example, cyanoacrylates, light-curable acrylics, polyurethanes, silicones, epoxies, and ultraviolet curable adhesives such as Henkel Loctite ®. In other embodiments, a combination of snap features and grooves may be provided in both distal end 1085 and the second tip portion 1092. Still other forms of fasteners may be used to affix the second tip portion 1092 to the anvil 1034. As can be seen in Fig. 50, the second tip portion 1092 has a downwardly extending substantially curved outer surface.

In various embodiments, the first tip portion 1088 and the second tip portion 1092 may be fabricated from a variety of different materials that may be identical to or different from the materials from which the staple cartridge 1012 and anvil 1034 are manufactured. For example, the first tip portion 1088 and the second tip portion 1092 may be manufactured from soft plastic, rubber, etc. The first tip portion 1088 and the second tip portion 1092 may be fabricated from the same or different materials.

In various embodiments, the first tip portion 1088 and the second tip portion 1092 are shaped such that their respective outer surfaces 1088', 1092' cooperate to substantially form a substantially blunt end effector nose generally designated as 1096 that, in one exemplary embodiment, has a paraboloid surface 1098 when the anvil 1034 is in the closed position as shown in Fig. 50. As used herein, the term "paraboloid surface" means a surface having parabolic sections parallel to a single coordinate axis and elliptic sections perpendicular to that axis. Those of ordinary skill in the art will appreciate that when employing various embodiments of the instrument 1000, as long as the surgeon can see one or the other of the first tip portion or second tip portion, the surgeon will know where the other tip portion is, even if it is behind tissue or other structures. In addition, the unique and novel tip configurations permit the surgeon to pass the anvil and/or channel around tissue without great risk of incidental trauma to adjacent tissues. Furthermore, when in the closed orientation as depicted in Figs. 49 and 50, these embodiments are particularly well suited for use as a dissector for separating and manipulating tissues.

The first tip portion and the second tip portion have been described and depicted in the Figures as being used in connection with a curved end effector. Those of ordinary skill in the art will readily appreciate, however, that the first and second tip portions may be used in connection with a variety of different end effector configurations such as linear endocutters and other types of end effectors. Thus, the first and second tip portions described above should not be limited solely to use in connection with curved endocutters/staplers.

As was described above, the first tip portion may be constructed for attachment to the distal end of a conventional staple cartridge or it may be integrally formed on the end of the staple cartridge. In still other embodiments, the first tip portion may be constructed for attachment to a distal end of the channel or it may be integrally formed on the distal end of the channel. Similarly, the second tip portion may be constructed for attachment to a conventional endocutter anvil or it may be integrally formed on the distal end of the anvil. In those applications wherein the first tip portion and/or second tip portion are fabricated separately from the cartridge and anvil, respectively, the tip portions may be supplied as a kit for retrofitting onto the cartridge and anvil by the end user. For example, in such arrangements, the tip portions may be presterilized and packaged and be configured to snap onto or otherwise attach to the staple cartridge and anvil or channel and anvil, whichever the case may be.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

## Claims

1. A surgical stapler, comprising:
a staple cartridge channel configured to receive a curved staple cartridge (912), the staple cartridge (912) configured to removably store staples therein;
an anvil (934) configured to deform said staples;
said staple cartridge (912); and
a curved piece of buttress material (971),
wherein said staple cartridge comprises:
a surface configured to receive said curved piece of buttress material thereon, wherein said surface includes a first end, a second end, and a first edge extending between said first end and said second end, and wherein said first edge is at least partially defined by a first radius of curvature; and **characterised in that** said staple cartridge comprises:
a first lip (977) which at least partially extends along said first edge, wherein said first lip (977) is configured to reduce relative movement between said piece of buttress material (971) and said surface,
wherein the first lip (977) is longer than the buttress material (971) and limits relative axial movement between the buttress material (971) and the staple cartridge (912).

2. The surgical stapler of Claim 1, further including a second lip which at least partially extends along said first end.

3. The surgical stapler of Claim 1, further including a second lip which at least partially extends along said second end.

4. The surgical stapler of Claim 1, wherein said surface further includes a second edge extending between said first end and said second end, wherein said second edge is at least partially defined by a second radius of curvature, and wherein said surgical stapler further includes a second lip which at least partially extends along said second edge.

5. The surgical stapler of Claim 1, further comprising adhesive for securing said piece of buttress material to said surface.

6. The surgical stapler of Claim 1, wherein said surgical stapler further comprises a second surface configured to receive a second piece of buttress material thereon and a second lip extending along the periphery of said second surface, and wherein said second lip is configured to reduce relative movement between said second piece of buttress material and said second surface.

7. The surgical stapler of Claim 5, wherein said anvil includes said second surface and said second lip, and wherein said anvil further comprises a third surface configured to receive a third piece of buttress material thereon and a third lip extending along the periphery of said third surface.

8. A method for processing an instrument for surgery, comprising:
obtaining the surgical stapler of Claim 1;
sterilizing said surgical stapler; and
storing said surgical stapler in a sterile container.

## Patentansprüche

1. Chirurgisches Klammergerät, umfassend:
einen Klammermagazinkanal, der dazu konfiguriert ist, ein gekrümmtes Klammermagazin (912) aufzunehmen, wobei das Klammermagazin (912) dazu konfiguriert ist, Klammern entfernbar darin zu lagern,
einen Amboss (934), der dazu konfiguriert ist, die Klammern zu deformieren,
das Klammermagazin (912) und
ein gekrümmtes Stück Versteifungsmaterial (971),
wobei das Klammermagazin Folgendes umfasst:
eine Fläche, die dazu konfiguriert ist, das gekrümmte Stück Versteifungsmaterial darauf aufzunehmen, wobei die Fläche ein erstes Ende, ein zweites Ende und einen sich zwischen dem ersten Ende und dem zweiten Ende erstreckenden ersten Rand aufweist, und wobei der erste Rand mindestens teilweise durch einen ersten Krümmungsradius definiert ist; und **dadurch gekennzeichnet, dass** das Klammermagazin Folgendes umfasst: eine erste Lippe (977), die sich mindestens teilweise entlang dem ersten Rand erstreckt, wobei die erste Lippe (977) dazu konfiguriert ist, Relativbewegung zwischen dem Stück Versteifungsmaterial (971) und der Fläche zu reduzieren, wobei die erste Lippe (977) länger als das Versteifungsmaterial (971) ist und axiale Relativbewegung zwischen dem Versteifungsmaterial (971) und dem Klammermagazin (912) begrenzt.

2. Chirurgisches Klammergerät nach Anspruch 1, das ferner eine zweite Lippe aufweist, die sich mindestens teilweise entlang dem ersten Ende erstreckt.

3. Chirurgisches Klammergerät nach Anspruch 1, das ferner eine zweite Lippe aufweist, die sich mindestens teilweise entlang dem zweiten Ende erstreckt.

4. Chirurgisches Klammergerät nach Anspruch 1, wobei die Fläche ferner einen sich zwischen dem ersten Ende und dem zweiten Ende erstreckenden zweiten Rand aufweist, wobei der zweite Rand mindestens teilweise durch einen zweiten Krümmungsradius definiert ist, und wobei das chirurgische Klammergerät ferner eine zweite Lippe aufweist, die sich mindestens teilweise entlang dem zweiten Rand erstreckt.

5. Chirurgisches Klammergerät nach Anspruch 1, ferner umfassend Klebstoff zum Befestigen des Versteifungsmaterialstücks an der Fläche.

6. Chirurgisches Klammergerät nach Anspruch 1, wobei das chirurgische Klammergerät ferner eine zweite Fläche, die dazu konfiguriert ist, ein zweites Stück Versteifungsmaterial darauf aufzunehmen, und eine zweite Lippe umfasst, die sich entlang dem Umfang der zweiten Fläche erstreckt, und wobei die zweite Lippe dazu konfiguriert ist, Relativbewegung zwischen dem zweiten Stück Versteifungsmaterial und der zweiten Fläche zu reduzieren.

7. Chirurgisches Klammergerät nach Anspruch 5, wobei der Amboss die zweite Fläche und die zweite Lippe aufweist und wobei der Amboss ferner eine dritte Fläche, die dazu konfiguriert ist, ein drittes Stück Versteifungsmaterial darauf aufzunehmen, und eine dritte Lippe umfasst, die sich entlang dem Umfang der dritten Fläche erstreckt.

8. Verfahren zur Bearbeitung eines Instruments für einen chirurgischen Eingriff, umfassend:
Erlangen des chirurgischen Klammergeräts nach Anspruch 1,
Sterilisieren des chirurgischen Klammergeräts und
Lagern des chirurgischen Klammergeräts in einem sterilen Behälter.

## Revendications

1. Agrafeuse chirurgicale, comprenant :
un canal de cartouche d'agrafes configuré pour recevoir une cartouche d'agrafes courbe (912), la cartouche d'agrafes (912) étant configurée pour stocker de manière amovible des agrafes à l'intérieur de celle-ci ;
une enclume (934) configurée pour déformer lesdites agrafes ;
ladite cartouche d'agrafes (912) ; et
un morceau courbe de matériau de renfort (971),
ladite cartouche d'agrafes comprenant :
une surface configurée pour recevoir ledit morceau courbe de matériau de renfort sur celle-ci, ladite surface comportant une première extrémité, une deuxième extrémité, et un premier bord s'étendant entre ladite première extrémité et ladite deuxième extrémité, et ledit premier bord étant au moins en partie défini par un premier rayon de courbure ; et **caractérisée en ce que** ladite cartouche d'agrafes comprend :
une première lèvre (977) qui s'étend au moins en partie le long dudit premier bord,
ladite première lèvre (977) étant configurée pour réduire le mouvement relatif entre ledit morceau de matériau de renfort (971) et ladite surface,
la première lèvre (977) étant plus longue que le matériau de renfort (971) et limitant le mouvement axial relatif entre le matériau de renfort (971) et la cartouche d'agrafes (912).

2. Agrafeuse chirurgicale selon la revendication 1, comportant en outre une deuxième lèvre qui s'étend au moins en partie le long de ladite première extrémité.

3. Agrafeuse chirurgicale selon la revendication 1, comportant en outre une deuxième lèvre qui s'étend au moins en partie le long de ladite deuxième extrémité.

4. Agrafeuse chirurgicale selon la revendication 1, dans laquelle ladite surface comporte en outre un deuxième bord s'étendant entre ladite première extrémité et ladite deuxième extrémité, ledit deuxième bord étant au moins en partie défini par un deuxième rayon de courbure, et ladite agrafeuse chirurgicale comportant en outre une deuxième lèvre qui s'étend au moins en partie le long dudit deuxième bord.

5. Agrafeuse chirurgicale selon la revendication 1, comprenant en outre un adhésif pour fixer ledit morceau de matériau de renfort à ladite surface.

6. Agrafeuse chirurgicale selon la revendication 1, ladite agrafeuse chirurgicale comprenant en outre une deuxième surface configurée pour recevoir un deuxième morceau de matériau de renfort sur celle-ci et une deuxième lèvre s'étendant le long de la périphérie de ladite deuxième surface, et ladite deuxième lèvre étant configurée pour réduire le mouvement relatif entre ledit deuxième morceau de matériau de renfort et ladite deuxième surface.

7. Agrafeuse chirurgicale selon la revendication 5, dans laquelle ladite enclume comporte ladite deuxième surface et ladite deuxième lèvre, et dans laquelle ladite enclume comprend en outre une troisième surface configurée pour recevoir un troisième morceau de matériau de renfort sur celle-ci et une troisième lèvre s'étendant le long de la périphérie de ladite troisième surface.

8. Procédé pour sous-traiter un instrument chirurgical, comprenant :
obtenir l'agrafeuse chirurgicale selon la revendication 1 ;
stériliser ladite agrafeuse chirurgicale ; et
stocker ladite agrafeuse chirurgicale dans un récipient stérile.
